Europäisches Patentamt

European Patent Office

Office européen des brevets

(11) Publication number: **0 320 148**
**A1**

## EUROPEAN PATENT APPLICATION

(21) Application number: **88311099.1**

(22) Date of filing: **23.11.88**

(51) Int. Cl.⁴: **C07K 13/00 , C12N 15/00 , C12P 21/02 , //C12N1/20, C12N5/00**

(30) Priority: **24.11.87 US 124896**
**17.11.88 US 271521**

(43) Date of publication of application:
**14.06.89 Bulletin 89/24**

(84) Designated Contracting States:
**AT BE CH DE ES FR GB GR IT LI LU NL SE**

(71) Applicant: **Amgen Inc.**
**1900 Oak Terrace Lane**
**Thousand Oaks, California 91320(US)**

(72) Inventor: **Arakawa, Tsutomu**
**4217 Minnesota**
**Thousand Oaks California 91360(US)**
Inventor: **Fox, Gary M.**
**11 Dorrit Court**
**Newbury Park California 91320(US)**

(74) Representative: **Brown, John David et al**
**FORRESTER & BOEHMERT**
**Widenmayerstrasse 4/I**
**D-8000 München 22(DE)**

(54) **Analogs of fibroblast growth factor.**

(57) Disclosed are recombinant basic fibroblast growth factor analogs possessing part or all of the primary structural conformation and one or more of the biological properties of a mammalian (e.g., human) basic fibroblast growth factor, wherein at least one of the cysteine residues of the naturally occurring basic fibroblast growth factor is replaced with a residue of a different amino acid. Also disclosed is a process for producing such analogs wherein a host cell is transformed or transfected with an exogenous DNA sequence encoding for the basic fibroblast growth factor analogs. Purification methods for the analogs are also disclosed as well as in vivo applications. Biologically active analogs of basic fibroblast growth factor are more stable and facilitate purification of basic fibroblast growth factor.

EP 0 320 148 A1

## ANALOGS OF FIBROBLAST GROWTH FACTOR

The present invention relates to analogs of basic fibroblast growth factor. In particular, the present invention refers to analogs of recombinant basic fibroblast growth factor ("r-bFGF") in an E. coli - (Escherichia coli) host strain and to polynucleotides encoding such factors. The following nomenclature is utilized: FGF = fibroblast growth factor; acidic FGF = aFGF; naturally occuring or natural FGF = n-FGF; human FGF = h-FGF; and bovine FGF = b-FGF; human basic FGF = h-bFGF; bovine basic FGF = b-bFGF; recombinant basic FGF = r-bFGF; recombinant human basic FGF = rh-bFGF; and recombinant bovine basic FGF = rb-bFGF.

### BACKGROUND

Fibroblast Growth Factor (FGF) was first described by Gospodarowicz, Nature, 249, 123 (1974) as an activity derived from bovine brain or pituitary tissue which was mitogenic for fibroblasts and endothelial cells. It was later noted that the primary mitogen from brain was different than that isolated from pituitary. These two factors were named acidic and basic FGF because they had similar if not identical biological activities but differed in their isoelectric points. Subsequently other endothelial cell mitogens were isolated from a number of tissues and tumors which are very similar or identical to basic FGF. Such factors include, for example, hepatoma-derived growth factor (Klagsbrun, et al, PNAS, 83, 2448-2452 (1986) and Lobb et al, J.Biol.Chem., 23, 6295-6299 (1984)), chondrosarcoma-derived growth factor (Shing et al, Science, 223, 1296-1299 (1984)), beta retina-derived growth factor (Baird et al, Biochemistry, 24, 7855-7860 (1985)), cartilage-derived growth factor (Sullivan and Klagsbrun, J. Biol. Chem., 260, 2399-2403 (1985)), astroglial growth factor 2 (Pettman et al, FEBS Lett., 189, 102-108), eye-derived growth factor (Courty et al, Biochimie, 67, 265-2698 (1985)), cationic hypothalamus-derived growth factor (Klagsbrun and Shing, PNAS, 82, 805-809 (1985)), class 2 and beta heparin-binding growth factors (Lobb and Fett, Biochemistry, 23, 6265-6299 (1984); Lobb et al, Biochem., 24, 4969-4973 (1985); Lobb et al, BBRC, 131, 586-592 (1985); Lobb et al, J.Biol.Chem., 261, 1924-1928 (1986)), and a component of macrophage-derived growth factor (Baird et al, BBRC, 126, 358-364 (1985)). All of the above factors share bFGF'S property of binding tightly to heparin and all are basic proteins. A similar group of heparin-binding factors, typified by aFGF, have also been found. These molecules elute from heparin at a lower sodium chloride concentration and have acidic isoelectric points. The heparin binding property of these factors has facilitated their purification, allowing isolation of sufficient protein for amino acid sequence analysis in several cases. Although the use of heparin has facilitated purification of FGF, the use of heparin in large scale purification procedures is undesirable because of the expense, possible contamination of product with heparin, and loss of yield due to irreversible binding to heparin. Acidic and basic FGF are probably derived from the same ancestral gene and are 55% homologous in amino acid sequence and have the same intron/exon structure. Southern blotting experiments suggest that there is only one gene each for acidic and basic FGF; differences between the molecules isolated from different tissues are probably due to post-translational processing. The range of biological activities of the two classes appears to be identical, although bFGF is about ten times more potent than aFGF in most bioassay systems.

Basic FGF is a single chain, non-glycosylated protein having a molecular weight of approximately 16,500. Basic FGF contains four cysteine residues, but the number of disulfide bonds, if any, is unknown. A primary translation product having 155 amino acids has been proposed for bFGF, but the major form found in pituitary tissue has 146 amino acids. Several molecular weight forms, differing in length at the N-terminus, have been isolated from different tissues, all of which appear to retain biological activity. Basic FGF is an extremely basic protein, with an isoelectric point of 9.6. Basic FGF binds avidly to heparin, eluting from heparin sepharose columns at around 1.6 M NaCl. The biological activity of bFGF is destroyed by heat (70° C) or by detergents. In the genome, coding sequences for this translation product are interrupted by two introns; the first splits codon 60 and the second separates codons 94 and 95. The size of the entire genomic coding region is not known, but it is at least 34 kb in length. The gene for bFGF is located on chromosome 4.

The first sequence data for bFGF was published by Bohlen et al PNAS, 81, 5364-5368, (1984) who determined the N-terminal 15 amino acids of material purified from bovine pituitary tissue. Subsequently, Esch et. al., PNAS, 82, 6507-6511, (1985) reported the complete sequence of bFGF from bovine pituitary and at the same time compared it with amino terminal sequence from aFGF. PCT patent application WO 86/07595 discloses the production of b-bFGF in E. coli. However, the reported yields of product are extremely low. Cloning of the gene for b-bFGF was first reported by Abraham et al Science, 233, 545-548, and a later paper by the same authors described the nucleotide sequence and genomic organization of h-bFGF (EMBO Journal, 5, 2523-2528 (1986)). Bovine and human bFGF are known to differ only by two amino acids.

Although highly purified preparations of bFGF have only recently been available for testing, many in vitro studies have been published using material of various states of purity. In these studies, bFGF has been shown to be a potent mitogen for a wide variety of cells of mesodermal origin and may be chemotactic for endothelial cells and fibroblasts. In addition, naturally occurring and tissue derived r-bFGF appears to induce neovascularization in both the rabbit cornea and chick chorioallantoic membrane assays, thus bFGF may be useful in accelerating the healing of wounds. Fourtanier et al, J.Inv.Derm., 87, 76-80 (1986) disclosed that a preparation derived from bovine retina was able to stimulate neovascularization and reepithelialization and to promote the healing of wounds in a guinea pig blister model. Davidson et al, J.Cell.Biol., 100, 1219-1227 (1985) have shown accelerated wound repair accompanied by increased granulation tissue and collagen accumulation to be induced by a bovine cartilage derived factor in a rat wound model system. Buntrock and coworkers, Exp.Path., 21, 46-53, and Exp.Path., 21, 62-67 (1982) have also reported increases in granulation tissue and neovascularization along with accelerated healing of wounds in rats using an extract of bovine brain tissue.

## SUMMARY OF THE INVENTION

The present invention relates to analogs of bFGF that are more stable than the naturally occurring bFGF and facilitate purification of r-bFGF. An analog of bFGF having an amino acid sequence comprising cysteine residues is provided, wherein at least one, and more preferably two, of said cysteine residues is replaced by a different amino acid residue.

The present invention also relates to a preferred process for producing bFGF analogs, said process comprising:

1) growing under suitable nutrient conditions, E. coli host cells transformed with a DNA plasmid vector wherein the DNA plasmid vector comprises a DNA sequence coding for E. coli host expression of a bFGF analog having part or all of the primary structural conformation of bFGF and one or more of the biological properties of human basic fibroblast growth factor wherein at least one cysteine residue is replaced by a residue of a different amino acid (hereinafter referred to as "bFGF analogs"), a regulated promoter sequence, and a temperature inducible copy number control gene;

2) isolating desired bFGF analogs of the expression of DNA sequences in said vector; and

3) purifying the desired bFGF analogs.

The present invention further relates methods for purifying E. coli derived r-bFGF analogs using non-heparin containing chromatographic systems.

DNA sequences coding for all or part of rh-bFGF analogs are provided. Such sequences preferably may include: (1) the incorporation of codons "preferred" for expression by selected E. coli host strains ("E. coli expression codons"); (2) the provision of sites of cleavage by restriction endonuclease enzymes; and/or, (3) the provision of additional initial, terminal or intermediate DNA sequences which facilitate construction of readily expressed vectors. The novel DNA sequences of the invention include sequences useful in securing expression in E. coli host cells of bFGF analogs.

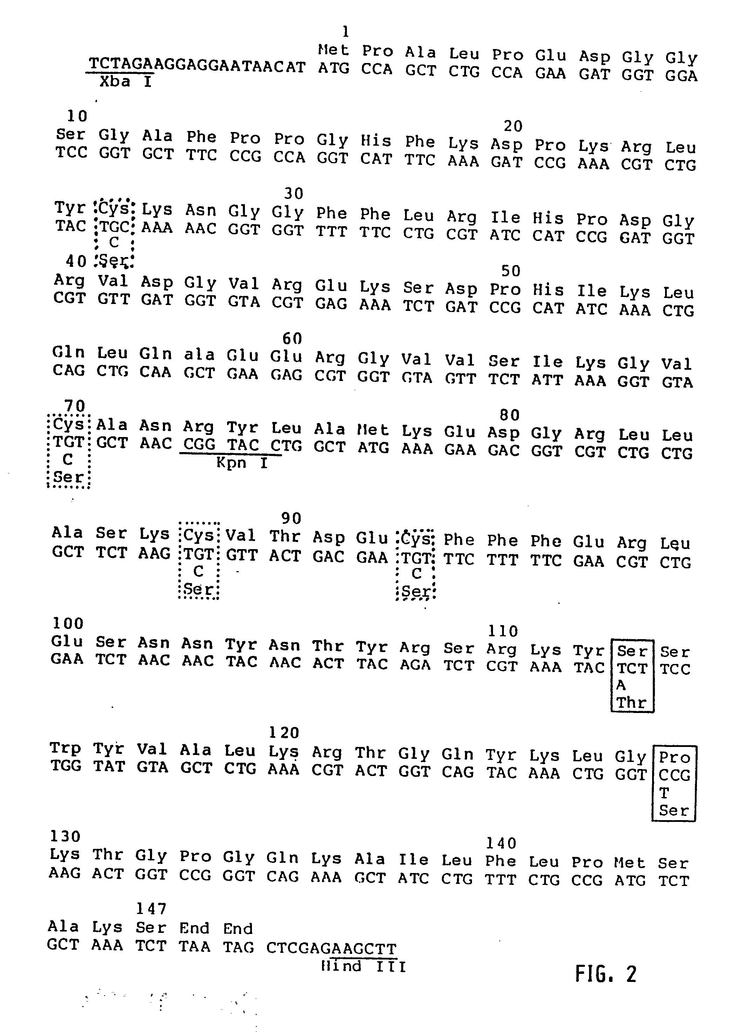

DNA sequences of the invention are specifically seen to comprise: (a) the DNA sequences set forth in Fig. 2 wherein at least one codon encoding a cysteine residue is replaced by a codon encoding a different amino acid residue (hereinafter "analog sequences"); (b) a DNA sequence which hybridizes to one of the analog sequences or to fragments thereof; and (c) a DNA sequence which, but for the degeneracy of the genetic code, would hybridize to one of the analog sequences. Specifically comprehended by part (c) are manufactured DNA sequences encoding bFGF analogs which DNA sequences may incorporate codons facilitating translation of messenger RNA in the preferred microbial hosts. Such manufactured sequences may readily be constructed according to the methods of Alton, et. al., PCT published application WO 83/04053.

Purified and isolated bFGF analogs having one or more of the biological properties (e.g., immunological properties and in vitro biological activity) and physical properties (e.g., molecular weight) of naturally-occurring bFGF including allelic variants thereof are described. These bFGF analogs may also be characterized by being the product of the preferred E. coli host expression of exogenous DNA sequences. The bFGF analogs of the present invention which are expressed from the preferred E. coli host cells may include an initial methionine amino acid residue (at position 1 as shown in Fig. 2). Alternatively, one or more of the terminal amino acid residues may be deleted from the DNA sequence while substantially retaining the biological activity of naturally occurring bFGF, as is known to those skilled in the art.

Various replicable cloning vehicles, expression vehicles and transformed E. coli cultures, all harboring the altered genetic information necessary to effect the production of E. coli derived bFGF analogs are also contemplated within the scope of the present invention.

Site-directed mutagenesis may be used to convert the gene for b-bFGF into one coding for rh-bFGF. Additionally, the bovine gene and human gene can be modified by site-directed mutagenesis to convert at least one of the cysteine residues.

Numerous aspects and advantages of the invention will be apparent to those skilled in the art upon consideration of the following detailed description which provides illustrations of the practice of the invention in its presently preferred embodiments.


Brief Description of the Drawings


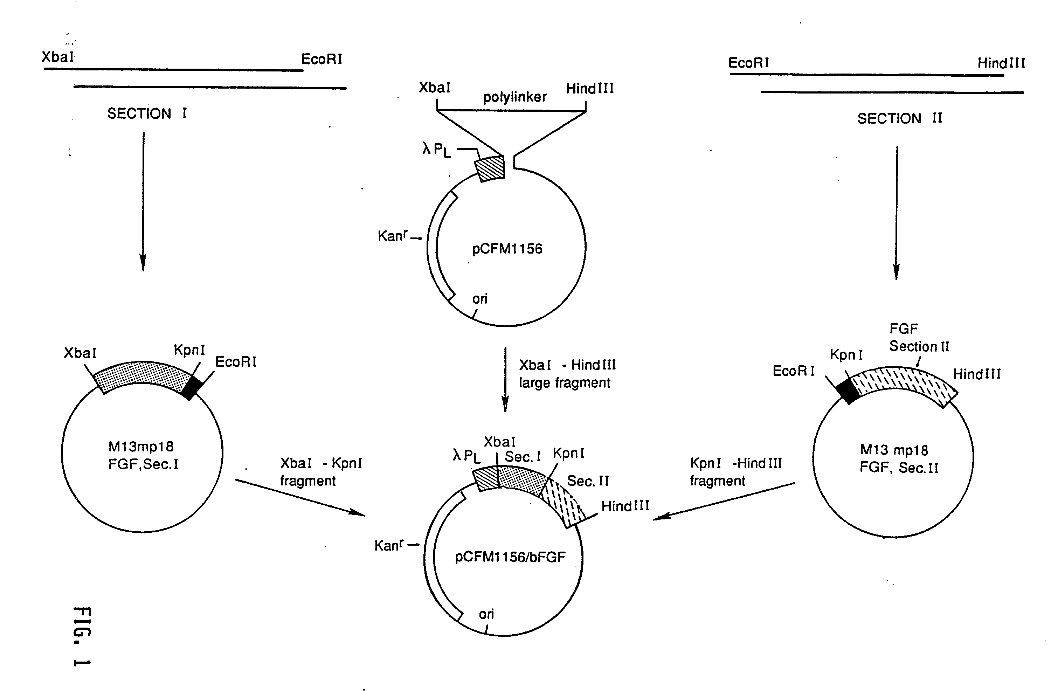

Fig. 1 is a diagrammatic representation of the bFGF gene assembly and cloning.

Fig. 2 is the nucleotide and amino acid sequences of r-bFGFs. The solid boxes outline the nucleotide and resultant amino acid changes which were produced by site-directed mutagenesis in order to convert the bovine gene to one coding for rh-bFGF. The dashed line boxes highlight the changes made to convert the bFGF gene into one coding for the bFGF analogs of the present invention.

Fig. 3 is a graph of the mitogenic activity of r-bFGFs on NIH3T3 cells. The mitogenic effect of rh-bFGF (●) and rb-bFGF (o) and recombinant human (ser-70,88) bFGF (x) on NIH3T3 cells is shown. The dose of r-bFGF is plotted against the percentage of maximal stimulation of DNA synthesis as measured by $^3$H thymidine uptake at that dose.

Fig. 4 is a photograph of a silver stained gel from sodium dodecyl sulfate polyacrylamide gel electrophoresis ("SDS-PAGE") of purified r-bFGFs.

Fig. 5 is a high pressure liquid chromatography (HPLC) profile of trypsin-digested native (top panel) and S-carboxymethylated (bottom panel) rb-bFGF. Arrows indicate the peptides which show major differences in the retention time upon S-carboxymethylation.

Fig. 6 is a circular dichroic spectrum of rb-bFGF in the far (left) and near (right) UV regions.

Fig. 7 is a graph of the mitogenic activity of the Ser-26,70,88,93 bFGF analog on NIH3T3 cells. The dose of r-bFGF is plotted against the percentage of maximal stimulation of DNA synthesis as measured by $^3$H-thymidine uptake at that dose.

Fig. 8 is a diagram for wounding and observation in the rabbit ear in vivo study of Example 16.


**DETAILED DESCRIPTION**


Novel bFGF analogs are provided in accordance with the present invention, wherein at least one, and more preferably two, of the cysteines found in natural bFGF are replaced with a different amino acid residue. These analogs have been found to exhibit a surprisingly marked increase in stability over the natural bFGF. It is believed that the more stable bFGF analogs of the present invention may also increase the efficacy of bFGF in wound healing treatments and in surgery.

Also provided by the present invention are manufactured or synthetic genes capable of directing synthesis, in selected microbial hosts (e.g., bacteria, yeast, and mammalian cells in culture), of the bFGF analogs.

Further comprehended by the present invention are pharmaceutical compositions comprising effective amounts of bFGF analogs of the invention together with suitable diluents, adjuvants and/or carriers useful in wound healing and surgical applications, including, but not limited to, the healing of surface wounds, bone healing, angiogenesis (formation of blood vessels, especially important in the healing of deep wounds), nerve regeneration, and organ generation and regeneration.

As used herein, the term "tissue-derived basic fibroblast growth factor" refers to basic fibroblast growth factor derived from tumors, eucaroytic cells maintained in culture, normal tissues and the like.

As employed herein, the term "manufactured" as applied to a DNA sequence or gene shall designate a product either totally chemically synthesized by assembly of nucleotide bases or derived from the biological replication of a product thus chemically synthesized. As such, the term is exclusive of products synthesized by cDNA methods of genomic cloning methodologies which involve starting materials which are initially of biological origin.

As employed herein, the term "synthesized" refers to site-directed mutagenesis or other alteration of a previously manufactured gene.

As herein described, the term "cysteine residues existing as free sulfhydryls" refers to cysteine residues that are not involved in forming disulfide bonds.

The E. coli derived recombinant bFGF was produced in accordance with the following general procedure:

The amino acid sequence of b-bFGF published by Esch et al PNAS, 82, 6507-6511 (1985) was used as a basis for manufacturing a bFGF gene for expression in E. coli. The nucleotide sequence of this manufactured gene includes codons most often used by E. coli and convenient restriction sites to be used for cloning purposes. Illustrative of a manufactured gene is the gene represented in Table I. Table I represents a manufactured gene for b-bFGF, while Table II represents a synthetic gene for h-bFGF. The nucleotide sequence of the manufactured gene for b-bFGF and the amino acid sequence from which it was derived is shown in Figure 2. The solid boxes outline the nucleotide and resultant amino acid changes which were produced by site-directed mutagenesis in order to convert the manufactured bovine gene to a synthetic gene coding for h-bFGF. The dashed line boxes highlight the subsequent changes made to convert the synthetic h-bFGF gene into analog sequences wherein one or more of the cysteine residues are replaced with serine residues. It is recognized that site-directed mutagenesis can be used to generate other b-bFGF analogs. In other words, other amino acids can be used to replace the cysteine residues. Generally, the amino acid selected to replace the cysteine residue is selected on the basis of its ability to create an analog which would retain a similiar structure but avoid dimer formation. Examples of such amino acids include serine, alanine, aspartic acid and asparagine. Other amino acids which may be substituted for cysteine will be apparent to those skilled in the art.

Oligonucleotides corresponding to both strands of the b-FGF gene were manufactured in overlapping sections and assembled into two larger sections by hybridization and subsequent ligation. The two larger sections were then cloned into an appropriate phage vector (i.e., M13mp18) for nucleotide sequence analysis. Such phage vectors are readily ascertained by one of ordinary skill in the art. Upon verification of the correct sequence, both sections were excised by restriction endonuclease digestion, gel isolated, and ligated into an appropriate expression vector. Expression of the bFGF gene encoded on the expression vector is regulated by a regulated promoter sequence and the temperature inducible copy number control genes located on the expression vector. The term "regulated promoters" as used herein, refers to $P_L$ promoters (e.g., promoters derived from a $\lambda$ phage) and foreshortened $P_L$ promoter. Expression vectors containing such regulated promoters and temperature inducible copy number control genes are described in European Patent Application No. 136,490. Growth of the expression vector containing the bFGF gene in an appropriate E. coli host strain yielded rb-bFGF. When the rb-bFGF-containing cells were lysed and subjected to

## TABLE I

Bovine basic Fibroblast Growth Factor/manufactured gene

```
           10                  30                  50
CTAGAAGGAGGAATAACATATGCCAGCTCTGCCAGAAGATGGTGGATCCGGTGCTTTCCC
GATCTTCCTCCTTATTGTATACGGTCGAGACGGTCTTCTACCACCTAGGCCACGAAAGGG


           70                  90                 110
GCCAGGTCATTTCAAAGATCCGAAACGTCTGTACTGCAAAAACGGTGGTTTTTTCCTGCG
CGGTCCAGTAAAGTTTCTAGGCTTTGCAGACATGACGTTTTTGCCACCAAAAAAGGACGC


          130                 150                 170
TATCCATCCGGATGGTCGTGTTGATGGTGTACGTGAGAAATCTGATCCGCATATCAAACT
ATAGGTAGGCCTACCAGCACAACTACCACATGCACTCTTTAGACTAGGCGTATAGTTTGA


          190                 210                 230
GCAGCTGCAAGCTGAAGAGCGTGGTGTAGTTTCTATTAAAGGTGTATGTGCTAACCGGTA
CGTCGACGTTCGACTTCTCGCACCACATCAAAGATAATTTCCACATACACGATTGGCCAT


          250                 270                 290
CCTGGCTATGAAAGAAGACGGTCGTCTGCTGGCTTCTAAGTGTGTTACTGACGAATGTTT
GGACCGATACTTTCTTCTGCCAGCAGACGACCGAAGATTCACACAATGACTGCTTACAAA


          310                 330                 350
CTTTTTCGAACGTCTGGAATCTAACAACTACAACACTTACAGATCTCGTAAATACTCTTC
GAAAAAGCTTGCAGACCTTAGATTGTTGATGTTGTGAATGTCTAGAGCATTTATGAGAAG


          370                 390                 410
CTGGTATGTAGCTCTGAAACGTACTGGTCAGTACAAACTGGGTCCGAAGACTGGTCCGGG
GACCATACATCGAGACTTTGCATGACCAGTCATGTTTGACCCAGGCTTCTGACCAGGCCC


          430                 450                 470
TCAGAAAGCTATCCTGTTTCTGCCGATGTCTGCTAAATCTTAATAGCTCGAGAAGCTT
AGTCTTTCGATAGGACAAAGACGGCTACAGACGATTTAGAATTATCGAGCTCTTCGAA
```

6

## TABLE II

### Human basic Fibroblast Growth Factor/synthetic gene

```
        10                  30                  50
CTAGAAGGAGGAATAACATATGCCAGCTCTGCCAGAAGATGGTGGATCCGGTGCTTTCCC
GATCTTCCTCCTTATTGTATACGGTCGAGACGGTCTTCTACCACCTAGGCCACGAAAGGG

        70                  90                  110
GCCAGGTCATTTCAAAGATCCGAAACGTCTGTACTGCAAAAACGGTGGTTTTTTCCTGCG
CGGTCCAGTAAAGTTTCTAGGCTTTGCAGACATGACGTTTTTGCCACCAAAAAAGGACGC

        130                 150                 170
TATCCATCCGGATGGTCGTGTTGATGGTGTACGTGAGAAATCTGATCCGCATATCAAACT
ATAGGTAGGCCTACCAGCACAACTACCACATGCACTCTTTAGACTAGGCGTATAGTTTGA

        190                 210                 230
GCAGCTGCAAGCTGAAGAGCGTGGTGTAGTTTCTATTAAAGGTGTATGTGCTAACCGGTA
CGTCGACGTTCGACTTCTCGCACCACATCAAAGATAATTTCCACATACACGATTGGCCAT

        250                 270                 290
CCTGGCTATGAAAGAAGACGGTCGTCTGCTGGCTTCTAAGTGTGTTACTGACGAATGTTT
GGACCGATACTTTCTTCTGCCAGCAGACGACCGAAGATTCACACAATGACTGCTTACAAA

        310                 330                 350
CTTTTTCGAACGTCTGGAATCTAACAACTACAACACTTACAGATCTCGTAAATACACTTC
GAAAAAGCTTGCAGACCTTAGATTGTTGATGTTGTGAATGTCTAGAGCATTTATGTGAAG

        370                 390                 410
CTGGTATGTAGCTCTGAAACGTACTGGTCAGTACAAACTGGGTTCGAAGACTGGTCCGGG
GACCATACATCGAGACTTTGCATGACCAGTCATGTTTGACCCAAGCTTCTGACCAGGCCC

        430                 450                 470
TCAGAAAGCTATCCTGTTTCTGCCGATGTCTGCTAAATCTTAATAGCTCGAGAAGCTT
AGTCTTTCGATAGGACAAAGACGGCTACAGACGATTTAGAATTATCGAGCTCTTCGAA
```

low speed centrifugation, about 30% to about 70% of rb-bFGF was found in soluble form in the supernatant fraction. Purification using non-heparin containing chromatographic systems, i.e., affinity chromatography resulted in a polypeptide product which was estimated to be at least 95% pure by polyacrylamide gel electrophoresis and contained virtually no endotoxin or DNA contamination.

Site-directed mutagenesis was employed to convert the bovine gene into one coding for human bFGF. The same purification scheme used for rb-bFGF was also used to purify rh-bFGF.

The mitogenic activity of the E. coli derived rb-bFGF, rh-bFGF, and the bFGF analogs of the present invention was measured using an in vitro mitogenic assay based on the increase in radiolabeled thymidine uptake by mouse 3T3 cells which accompanies increased DNA synthesis during cell division.

Oligonucleotide site-directed mutagenesis was used to modify the rh-bFGF gene so that sequences which coded for some or all of the cysteine residues would now encode for different amino acid residues. The bFGF analogs of the present invention were surprisingly found to impart markedly greater stability to the bFGF molecule, while not detracting from bFGF activity.

The following examples serve to further illustrate the embodiments of the present invention. The term "OD", as used in these examples, refers to optical density units at 600 nm.

## EXAMPLE 1

### Preparation of a Manufactured Gene Encoding b-bFGF

This example relates to the preparation of a manufactured gene encoding b-bFGF wherein E. coli expression preference codons are included. The protocol employed to prepare the manufactured gene encoding a b-bFGF product is generally described in the disclosure of Alton, et al, PCT Publication No. WO83/04053 which is incorporated by reference herein. The genes were designed for initial assembly of component oligonucleotides into multiple duplexes which, in turn, were assembled into 2 discrete sections. These sections were designed for ready amplification and, upon removal from the amplification system, could be assembled sequentially or through a multiple fragment ligation in a suitable expression vector.

### Assembly of Section I of Fibroblast Growth Factor

200 pm of each of the 16 oligomers required for assembly of section I represented in Table III were measured into eppendorf tubes and dried on a speed vacuum pump. 320 μl of a kinase mix was prepared which contained 32 μl of 10x ligation buffer (50 M HEPES, pH 7.6), 0.7 μl of 10 mM adensosine triphosphate (ATP), 1 μl of $3 \times 10^7$ counts/minute/μl of radiolabelled ATP, and 266 μl of water. The reagents were combined in a tube of kinase (Boehringer Mannheim, Ingelheim, West Germany) which contained 20 μl of the kinase enzyme at a concentration of 10 units/μl. This kinase mix was aliquoted into each of tubes 2-15 containing oligonucleotides 2-15, respectively. Tubes containing oligonucleotides 1 and 16 received ligation buffer only. Tubes containing 2-15 were incubated at 37° C for 45 minutes. At the end of that time period, 1/4 μl aliquots from each tube were spotted onto DE81 paper chromatography strips (Whatman, Maidstone, U.K.) eluted with 0.35 M ammonium formate and analyzed on a liquid scintillation counter. Liquid scintillation analysis showed that more than 1/2 of the counts were at the

## TABLE III

FGF OLIGOMERS, SECTION I

1) 5′    CTAGAAGGAGGAATAACATATGCCAGCTCT    3′

2) 5′    GCCAGAAGATGGTGGATCCGGTGCTTTCCC    3′

3) 5′    GCCAGGTCATTTCAAAGATCCGAAACGTCTG    3′

4) 5′    TACTGCAAAAACGGTGGTTTTTTCCTGCGTA    3′

5) 5′    TCCATCCGGATGGTCGTGTTGATGGTGTAC    3′

6) 5′    GTGAGAAATCTGATCCGCATATCAAACTGCA    3′

7) 5′    GCTGCAAGCTGAAGAGCGTGGTGTAGTTT    3′

8) 5′    CTATTAAAGGTGTATGTGCTAACCGGTACCTG    3′

9) 5′    CTGGCAGAGCTGGCATATGTTATTCCTCCTT    3′

10) 5′    TGGCGGGAAAGCACCGGATCCACCATCTT    3′

11) 5′    AGTACAGACGTTTCGGATCTTTGAAATGACC    3′

12) 5′    ATGGATACGCAGGAAAAAACCACCGTTTTTGC    3′

13) 5′    TCACGTACACCATCAACACGACCATCCGG    3′

14) 5′    CAGCTGCAGTTTGATATGCGGATCAGATTTC    3′

15) 5′    ATAGAAACTACACCACGCTCTTCAGCTTG    3′

16) 5′    AATTCAGGTACCGGTTAGCACATACACCTTTA    3′

origin. As a result, 2 μl of 10 mM ATP were added to each of the tubes containing 2-15 oligonucleotides, and the tubes incubated an additional 45 minutes at 37°C. At the end of this time period, all tubes were boiled for 10 minutes then centrifuged and combined into duplexes. This was done by adding the contents of tube 9 to tube 1 (duplex #1), tube 10 to tube 2 (duplex #2), tube 11 to tube 3 (duplex #3), tube 12 to tube 4 (duplex #4), tube 13 to tube 5 (duplex #5), tube 14 to tube 6 (duplex #6), tube 15 to tube 7 (duplex #7), and tube 16 to tube 8 (duplex # 8). These eight mixtures of oligonucleotides were then boiled and slow cooled to room temperature to allow formation of the duplexes. The duplexes were then combined so that duplexes #1 and #2 were combined (tetramer 1 + 2), duplexes #3 and #4 (tetramer 3 + 4) were combined, duplexes #5 and #6 (tetramer 5 + 6) were combined, and finally duplexes #7 and #8 (tetramer 7 + 8) were combined. To each of these tubes, now containing tetramers, 2 μl of 10 mM ATP and 2 μl of T4 DNA ligase from Boehringer Mannheim were added. These ligation mixtures were then incubated for 10 minutes at 37°C and then at room temperature for 1 hour. At this point, the tetrameric mixtures were pooled again so that the tetramers 1 + 2 and 3 + 4 were combined together and tetramers 5 + 6 and 7 + 8 were combined together. To each of the two resulting ligation mixtures was added an additional 2 μl of 10 mM ATP and 2 μl of T4 DNA ligase. The mixtures were incubated for 10 minutes at 37°C and then at room temperature for 1 hour. Finally the entire ligation was pooled together, that is, the contents of both tubes were added together, an additional 8 μl of ligase were added to the ligation mixture, and the entire mixture incubated for 10 minutes at 37°C and then overnight at 4°C. Following overnight ligation, one 10 µl aliquot was analyzed on an 8% polyacrylamide gel made up with 7 M urea. A band could be discerned adjacent to the 242 base pair HpaII marker, which indicated that the ligation was complete. An 8% polyacrylamide gel was made which also contained 7 M urea. The ligation mix was ethanol precipitated, dried and then taken up in 80 µl of 80% formamide. Half of this ligation mix was then loaded onto the preparative gel adjacent to a lane containing HpaII cut PBR322 markers. The gel was run until the xylene cyanol dye marker had reached the bottom of the gel. The gel was then removed from the electrophoresis apparatus and placed in a film cassette next to a piece of Kodak X-Ray film. The bands were visualized by developing the film, and a gel slice just above the HpaII 242 marker on the adjacent lane removed. This slice contained the 244 base pair band expected for the completely ligated section I of fibroblast growth factor. This gel slice was extruded through a syringe into an eppendorf tube, and covered with Maxam Gilbert Gel Elution solution and incubated overnight at 37°C. The contents of the tube were then filtered through a glass fiber filter placed in a syringe barrel and the supernatant was extracted three times with N-butanol and ethanol precipitated. The dried pellet was then taken up in 200 µl of a solution of 10 mM Tris-HCl and 1 mM ethylene diamine tetraacetic acid (EDTA), and reprecipitated with ethanol after removing the polyacrylamide residue which was centrifuged in the bottom of the tube. The ethanol precipitated sample contained about 37,000 counts per minute which corresponded to about 1.5 pm of duplex based upon the radioactivity corresponding to the oligomers required for this ligation. These 1.5 pm were then dissolved in 20 µl of ligation buffer containing $3 \times 10^7$ counts per minute of radiolabelled ATP. A 1/4 µl aliquot was removed and spotted on a DE81 strip. Then, I µl of kinase was added and the tube was incubated at

## TABLE IV

FGF OLIGOMERS, SECTION II

17) 5' AATTCGGTACCTGGCTATGAAAGAAGACGGTCGTCTGCTGG 3'

18) 5' CTTCTAAGTGTGTTACTGACGAATGTTTCTTTTTCGAACG 3'

19) 5' TCTGGAATCTAACAACTACAACACTTACAGATCTCGTAAA 3'

20) 5' TACTCTTCCTGGTATGTAGCTCTGAAACGTACTGGTCAGT 3'

21) 5' ACAAACTGGGTCCGAAGACTGGTCCGGGTCAGAAAGCTATCC 3'

22) 5' TGTTTCTGCCGATGTCTGCTAAATCTTAATAGCTCGAGA 3'

23) 5' GAAGCCAGCAGACGACCGTCTTCTTTCATAGCCAGGTACCG 3'

24) 5' CAGACGTTCGAAAAAGAAACATTCGTCAGTAACACACTTA 3'

25) 5' ATGATTTACGAGATCTGTAAGTGTTGTAGTTGTTAGATTC 3'

26) 5' TTGTACTGACCAGTACGTTTCAGAGCTACATACCAGGAAG 3'

27) 5' AAACAGGATAGCTTTCTGACCCGGACCAGTCTTCGGACCCAGT 3'

28) 5' AGCTTCTCGAGCTATTAAGATTTAGCAGACATCGGCAG 3'

37°C for 45 minutes. At this point, 1/4 µl was removed from the tube and spotted on a second DE81 strip. Both strips were then eluted with 0.35 M ammonium formate and then cut into sections and counted on a liquid scintillation counter. The before and after strips clearly showed that counts were incorporated at the origin, therefore, the kination reaction was driven to completion by the addition of with 1 μl of 10 mm ATP and incubation for 30 minutes longer at 37°C. Then the kination mixture was boiled for 5 minutes and slow cooled to room temperature.

## Assembly of Section II of Fibroblast Growth Factor

Fibroblast Growth Factor Section II was assembled in a similar manner. 200 pm of each of the 12 oligonucleotides represented in Table IV were measured into eppendorf tubes and speed-vacuumed to dryness. The drying was repeated with 100 μl of 80% ethanol. A kinase mix was prepared which contained 24 μl of 10x ligation buffer, 2 μl of radiolabelled ATP ($1.5 \times 10^7$ counts/minute/ul), 0.5 μl of 10 mM ATP, 20 μl of kinase and 193 μl of water giving the total volume of 240 μl in the kinase mix. 20 μl of this mixture were added to each of the tubes, containing oligonucleotides 18-27, respectively, to be kinased. Tubes containing oligonucleotides 17 and 28 were given ligation buffer only. The tubes were then incubated for 45 minutes at 37°C at which time a 1/4 μl aliquot was removed from each tube and spotted onto DE81 strips. The DE81 strips were then eluted with 0.35 M ammonium formate and checked with a liquid scintillation counter to determine the number of counts at the origin. The analysis showed that the kination was proceeding. At this point, 1 μl of 10 mM ATP was added to each tube and the tubes were incubated for an additional 45 minutes at 37°C. The tubes were boiled for 5 minutes and then centrifuged and combined to form duplexes. Oligonucleotide #23 was combined with #17 (duplex #17), #24 with #18 (duplex #18), #25 with #19 (duplex #19), #26 with #20 (duplex #20), #27 with #21 (duplex #21), and #28 with #22 (duplex #22). These duplex mixtures were then boiled and slow cooled to room temperature over a period of 1 hour. The duplexes were then combined to form tetramers. Duplexes 17 + 18 were combined to form tetramer 17, duplexes 19 + 20 were combined to form tetramer 19, and duplexes 21 + 22 were combined to form tetramer 21. These were annealed at 37°C for 10 minutes. To each tetrameric mixture were added 2 μl of 10 mM ATP and 2 μl of T4 DNA ligase. The 3 ligations were incubated overnight at 4°C. At this point 4 μl aliquots were removed from each of the three tubes containing the tetramers and run on a 10% polyacrylamide gel made with 7 M urea. Autoradiography of the gel showed that the ligation was proceeding. Tetramers 17 and 19 were pooled and 4 μl of ligase were added along with 4 μl of 10 mM ATP. The 8 piece ligation was then incubated at 37°C for 15 minutes and then at 4°C at 6 hours before adding the last tetramer to the ligation mixture. At this point tetramer 21 was added to the octameric ligation mixture and the entire ligation was incubated at 37°C for 15 minutes. 5 μl of ligase and 5 μl of 10 mM ATP were added, and the resultant mixture incubated at 37°C for 15 minutes. 5 μl of ligase and 5 μl of 10 mM ATP were again added to the ligation mixture, and the entire ligation incubated overnight at 4°C. A check of the full ligation on an 8% polyacrylamide gel made with 7 M urea showed a prominent band at 242 base pairs as expected. The ligation mix was phenol extracted and ethanol precipitated before loading onto a prep gel. 1/2 of the ligation mix was loaded onto an 8% 7 M urea gel and the 242 base pair product was visualized by autoradiography, removed, and purified as described for section I of the bFGF.

## EXAMPLE 2

## Cloning and Expression of rb-bFGF

The b-bFGF gene was synthesized in two sections as described in Example 1. Each section was cloned into M13mp18 for sequence verification before assembly into an expression vector, pCFM1156. In preparation for the cloning of Section I, M13mp18 was digested with a threefold excess of restriction enzymes EcoRI and XbaI for 2 hrs. The reaction was terminated by extraction with an equal volume of phenol followed by extraction with chloroform and precipitation with 2.5 volumes of ethanol. The DNA pellet was washed with ethanol, dried under vacuum and dissolved in 10 mM Tris, 0.1 mM EDTA, pH 7.4. Ligation was carried out by incubation of 0.06 pmole of the M13mp18 vector prepared as described with 0.3 pmole of the synthetic FGF Section I in 50 mM tris (pH 7.4), 10 mM $MgCl_2$, 10 mM dithiothreitol (DTT), 1 mM spermidine, 1 mM ATP, 100 μg/ml bovine serum albumin (BSA), and 1 unit T4 ligase for 4 hours at 14°C. E. coli JM109 host cells were made competent by centrifugation of cells from an exponentially growing culture, suspension in ice-cold 50 mM $CaCl_2$ at a concentration of 1.2 OD/ml for 20 minutes, followed by recentrifugation and resuspension of the cells in the same solution at a concentration of 12 OD/ml. Aliquots of the ligation mixture (0.1-10 μl) were added to 200 μl aliquots of the competent host cells and allowed to stand on ice for 40 minutes. The contents of each tube was then added to 200 μl of fresh E. coli JM109, 3 ml of molten 0.7% Luria agar containing 10 μl of 100 mM isopropyl-β-D-thio-galactopyranoside (IPTG) and 50 μl of 2% 5-bromo-4-chloro-3-indolyl-β-D-galactopyranoside (X-Gal). This mixture was plated on a Luria plate and incubated overnight at 37° C. Four of the resulting clear plaques were picked from the plates and grown in 10 ml cultures using JM109 as the host strain. Single strand phage DNA was prepared from these cultures and sequenced by the dideoxy method using M13 universal primer. One of these four DNA's had the desired sequence and was saved for assembly of the rb-bFGF gene into the expression vector.

Section II of the manufactured b-bFGF gene was cloned into M13mp18 for sequencing using the same method as for section I. In this case, the M13mp18 vector was digested with EcoRI and HindIII (3-fold excess) in order to accommodate the sticky ends of section II. For the ligation, 0.025 pmole of M13mp18 vector was mixed with 0.075 pmole of the phosphorylated synthetic FGF section II and incubated 4 hours at 14° C as before. Transformation using the same CaCl₂ method resulted in clear plaques as for Section I, but since a high background of clear plaques was present on the control plates, further selection by hybridization was carried out. Several plaques were grown using JM109 host as described before and supernatants containing phage DNA were dotted on nitrocellulose filters. Oligonucleotides 18 and 24 used in the synthesis of Section II were radiolabeled with ³²P-ATP using polynucleotide kinase and were used to probe these filters. Two positive-screening clones were selected and sequenced as before. One of these clones had the expected sequence and was used in the assembly of the gene into pCFM1156.

## EXAMPLE 3

Assembly of rb-bFGF Gene in the Expression Vector pCFM1156

Double-stranded replicative form DNA for the section I and II M13 clones was prepared. 500 ml cultures of each phage in JM109 host were grown and the cells harvested by centrifugation. Cells were then resuspended in 15% sucrose, 0.05 M tris, 0.05 M EDTA, and 1 mg/ml lysozyme and incubated on ice for 25 minutes. RNAse was added to 0.1 mg/ml and incubation on ice continued for 10 more minutes. An equal volume of 0.1% triton X-100, 50 mM tris, 50 mM EDTA was added and incubation on ice continued for another 10 minutes. These lysates were then centrifuged for 60 minutes at 39000G and the clear supernatant saved. Ethidium bromide was added to 1 mg/ml and cesium chloride was added to give a density of 1.55 g/ml. This solution was centrifuged for 18 hours at 45,000 rpm in a VTi-50 rotor in order to reach equilibrium. The supercoil DNA band from each tube was visualized by UV light and collected with a syringe. The ethidium bromide was quickly removed by extraction with butanol and the CsCl was removed by extensive dialysis against 10 mM tris, 0.1 mM EDTA. DNA stocks prepared in this way were used for further cloning.

Although numerous vectors may be employed to express this DNA, an expression vector having a regulated promoter and temperature inducible copy number control gene is preferred in order to maximize product yields. The expression plasmid pCFM1156 used in this example may readily be constructed from a plasmid pCFM836, the construction of which is described in published European Patent Application No. 136,490. The pCFM836 is first cut with NdeI and then blunt-ended with T4 polymerase such that both existing NdeI sites are destroyed. Next, the vector is digested with ClaI and SacII to remove an existing polylinker before ligation to a substitute polylinker as illustrated in Table V. This substitute polylinker may be constructed according to the procedure of Alton, et. al., supra. Control of expression in the expression pCFM1156 plasmid is by means of a foreshortened lambda P_L promoter, which itself may be under the control of a C_{I857} repressor gene (such as is provided in E. coli strain K12 ΔHtrp).

The expression vector pCFM1156 was digested (2-fold excess) with XbaI and HindIII in preparation for ligation with FGF sections I and II. Section I and II DNA stocks, prepared as described above, were digested (2-fold excess) with either XbaI and KpnI (section I) or KpnI and HindIII (section II). All three digests were loaded onto a 1.2% low melting agarose gel made in 50 mM tris-acetate and electrophoresed for 3 hours at 60 volts. The gel was stained with 1 μg/ml ethidium bromide solution and the bands were visualized under UV light. The linearized vector band and the FGF section I and II bands were excised from the gel with a scalpel, placed in separate tubes and melted at 70° C for 15 minutes. Five microliters of the molten gel containing linearized vector was added to 10 μl each of the molten gel containing sections I and II and the mixture equilibrated to 37° C. The molten gel was mixed quickly with an equal volume of ice-cold 2X ligase buffer containing 2 mM ATP, and 0.5 unit T4 ligase and incubated overnight at 14° C. Aliquots of this ligation mix were transformed into frozen competent E. coli FM6 host strain using a transformation protocol described by Hanahan, J. Mol. Biol. 166, 557-580, (1983), grown 2.5 hours to allow expression of kanamycin resistance, and plated on Luria plates containing 20 μg/ml kanamycin. Plates were incubated at 28° C overnight. Colonies were replica plated onto nitrocellulose filters and the master plate was saved. Colonies on the filters were grown to about 1 mm diameter at 28° C and then placed at 37° C overnight to increase plasmid copy number. The filters were screened by hybridization with radiolabeled oligonucleotide 18 (from gene synthesis) at 65° C in 6X standar saline citrate buffer (SSC, 0.15 M NaCl, 0.015 M Na citrate). Of the twenty-five positive clones obtained, four were selected and grown in 500 ml cultures. Replicative form DNA was prepared as

## TABLE V

```
  1  ATCGATTTGATTCTAGAAGGAGGAATAACATATGGTTAACGCGTTGGAATTCGGTACCAT
     TAGCTAAACTAAGATCTTCCTCCTTATTGTATACCAATTGCGCAACCTTAAGCCATGGTA

     1 ClaI, 12 XbaI, 29 NdeI, 35 HincII, HpaI, 39 MluI, 47  EcoRII,
       53 HgiCI KpnI, 57 NcoI StyI,

 61  GGAAGCTTACTCGAGGATCCGCGGATAAATAAGTAACGATCC
     CCTTCGAATGAGCTCCTAGGCGCCTATTTATTCATTGCTAGG

     63 HindIII, 70 AvaI XhoI, 75 BamHI Xho2, 79 Sac2,
```

described for sections I and II using a cleared lysate procedure followed by CsCl equilibrium density gradient centrifugation. These four clones were sequenced directly using the expression vector's double stranded form as a template for the dideoxy sequencing reactions, and all four clones were found to have the correct sequence. One of these clones was chosen to be utilized in the expression of rb-bFGF and is hereinafter referred to as pCFM1156/bFGF. The DNA sequence for the rb-bFGF gene thus constructed in the pCFM1156/bFGF vector is represented in Table I.

## EXAMPLE 4

Expression and Purification of rb-bFGF

Expression

An overnight culture of the production strain was grown at 28° C in Luria broth (Luria broth: bactotryptone 10 g/l, yeast extract 5 g/l, NaCl 5 g/l) containing 20 μg/ml kanamycin, and was used to inoculate an 8-liter fermentation batch. The 8-liter batch media contained 40 g yeast extract, 40 g glucose, 10 g sodium chloride, and appropriate buffer salts, vitamin solution, and trace metals. A dual feed protocol was used. The initial feed (1 liter) contained 450 g glucose plus appropriate vitamins and salts. After growth to 20 OD a second feed was begun at a rate of 200 ml/hour and the temperature was shifted to 42° C. This feed solution contained 200 g/l bacto-tryptone, 100 g/liter yeast extract, and 100 g/l glucose. Growth was continued for 6 hours at 42° C with the cell concentration reaching 50 OD at harvest.

Purification

E. coli cells were broken in water by a Gaulin homogenizer, and centrifuged at 4.2 K for 40 min with a J6B centrifuge. When analyzed by SDS-PAGE, rb-bFGF appeared in both pellet and supernatant, the protein in the supernatant being 60-70%. The pellet was, therefore, discarded and the supernatant was purified using ion exchange chromatography. The supernatant, after titration to pH 7.4 with Tris-base, was made l mM DTT and mixed with a carboxymethyl cellulose-Sehparose® resin (CM-Sepharose®, Pharmacia, Uppsala, Sweden) equilibrated with 40 mM Tris-HCl/1 mM DTT/pH 7.4. The resin was then washed batch-wise with the same buffer, and eluted column-wise with a linear NaCl gradient from 0 to 0.7 M. A single peak around 0.5 M was pooled based on SDS-PAGE analysis. The pool was titrated to pH 8.2 with Tris-base, diluted three-fold with cold water, and loaded onto a CM-Sepharose in 40 mM Tris-HCl/1 mM DTT/pH 8.2. The column was washed with 0.15 M NaCl and eluted with a linear NaCl gradient of 0.15 to 0.5 M. A major peak between two small peaks was pooled and found to be approximately 95% pure with a small amount of dimer when analyzed by non-reducing SDS-PAGE.

The pool was immediately titrated to approximately pH 5 with 1 M sodium acetate/pH 4 to prevent the rb-bFGF product from oxidation and then directly loaded on a Sephadex G-75 column in 20 mM Na citrate/0.1M NaCl/pH 5, resulting in a single peak eluting between a shoulder (corresponding to dimer) and a peak for small compounds (such as DTT). The fractions in the peak were pooled and stored at 4°C, -20°C or lyophilized. The yield in this gel filtration was nearly 100%. Approximately 160 mg of rb-bFGF was obtained from 560 g of cell paste.


## TABLE VI


### Human basic Fibroblast Growth Factor/peptide sequence

```
         1                            10                              20
MetProAlaLeuProGluAspGlyGlySerGlyAlaPheProProGlyHisPheLysAsp

                                     30                              40
ProLysArgLeuTyrCysLysAsnGlyGlyPhePheLeuArgIleHisProAspGlyArg

                                     50                              60
ValAspGlyValArgGluLysSerAspProHisIleLysLeuGlnLeuGlnAlaGluGlu

                                     70                              80
ArgGlyValValSerIleLysGlyValCysAlaAsnArgTyrLeuAlaMetLysGluAsp

                                     90                             100
GlyArgLeuLeuAlaSerLysCysValThrAspGluCysPhePhePheGluArgLeuGlu

                                    110                             120
SerAsnAsnTyrAsnThrTyrArgSerArgLysTyrThrSerTrpTyrValAlaLeuLys

                                    130                             140
ArgThrGlyGlnTyrLysLeuGlySerLysThrGlyProGlyGlnLysAlaIleLeuPhe

         146
LeuProMetSerAlaLysSer
```


EXAMPLE 5


Characterization of rb-bFGF

The activity of rb-bFGF was examined in $^3$H-thymidine incorporation on 3T3 cells. All the preparations, stored at 4°C, and -20°C and lyophilized, showed a dose-dependent activity with a protein concentration from 20-210 pg/ml for half maximal activity depending on the particular strain of 3T3 cells utilized in the assay.

| General Characteristics of the Final Product | |
| --- | --- |
| 280/260 ratio | ~2.0 |
| LAL endotoxin assay | <0.6 EU/ml (0.623 FGF mg) |
| DNA | <20 pg/ml (0.623 FGF mg) |
| Extinction coefficient (278 nm) | 1.3 for 0.1% protein |
| Purity | >95% |

Stability

When a rb-bFGF preparation was incubated at 4°C at different pH values, rb-bFGF showed formation of polymers composed of more than one bFGF molecule at pH ≥ 6.0 due to inter-chain disulfide bonds and degradation at pH ≤ 4.0 due to acid instability of Asp-Pro bond. Based on this information, the pH 5 buffer was selected. This stability data suggests that free sulfhydryl groups present in the final product tend to form inter-chain, rather than intra-chain, disulfide bonds. The rb-bFGF preparation is apparently monomeric when determined by a Sephadex G-75 gel filtration.

Example 6

Structural Characterization of rb-bFGF

Purified rb-bFGF protein prepared as described in Example 4 was examined for conformational analyses using sulfhydryl titration, circular dichroism (CD) and gel filtration. The cysteine residues in the molecule were also studied by peptide mapping and sequence analysis.

Methods

Ultra-pure urea was obtained from Schwarz/Mann (Cleveland, Ohio). 5,5'-dithiobis-(2-nitrobenzoic acid) (DTNB) was obtained from Sigma Chemical Company (St. Louis, Missouri). Recombinant b-bFGF was purified from E. coli cells as described in Example 4.

Into 12 ml of rb-bFGF at 0.626 mg/ml in 20 mM Na citrate, 0.1 M NaCl, pH 5.0 was added 1.5 ml of 0.5 M iodoacetacetic acid in 1 M Tris-HCl, pH 8.2 (final 55 mM), then titrated to pH 8.2 with saturated Tris-base. The mixture was allowed to stand for 12 hours at room temperature, then dialyzed against cold 20 mM sodium citrate, 0.1 M NaCl, pH 5.0. After exhaustive dialysis, the protein solution was concentrated and centrifuged to remove trace amounts of precipitates. Titration of sulfhydryl groups was carried out using DTNB essentially according to Habeeb, A.F.S.A. 25 Methods. Enzymol. 457-465 (1972).

Gel filtration was carried out on a Sephadex® G-75 column (2.5 x 110 cm, Pharmacia, Uppsala, Sweden) in 20 mM sodium citrate, 0.1 M NaCl, pH 5.0 at a flow rate of 1 ml/min. The column was calibrated with myoglobin (17000) and bovine serum albumin (68000).

Protein concentration was determined spectrophotometrically.

UV absorbance spectra were determined on a Hewlett-Packard Model 8451A diode spectrophotometer. Circular dichroic spectra were determined at room temperature on a Jasco Model J-500C spectropolarimeter equipped with an Oki If 800 model 30 computer. Measurements were carried out at room temperature with cuvettes of 1 and 0.02 cm for near and far UV ranges, respectively. The data were expressed as the mean residue ellipticity, [θ], calculated using the mean residue weight of 112 for bFGF.

Trypsin digests were performed for 4 hours at 37°C in 0.1 M ammonium bicarbonate, pH 8.0, using tosylphenylalanyl chloromethyl ketone-treated trypsin (Cooper Biomedical, Malvern, Pennsylvania). The rb-bFGF concentration was 0.5 mg/ml, with the enzyme to substrate ratio being 1:50.

Peptide mapping was done by reverse phase HPLC chromatography using a Vydac C4 column (214TP54) (Vydac, Hesperia, California) run at a flow rate of 0.8 ml/min. The gradient ran from 0% buffer A (0.1% trifluoroacetic acid (TFA)/water) to 50% buffer B (0.1% TFA/90% acetonitrile/water) with a linear gradient of 90 minutes. Samples were injected under a Wisp® (Waters Corporation, Milford, Massachusetts) autosampler control. Fractions were collected by automatic peak detection monitoring at 220 nm.

Sequence analysis was performed on instruments from Applied Biosystems (Foster City, CA). Models 470A and 477A were used. In the 470A instrument, a modified R2 reagent was used (Lai, 163 Anal. Chim. Acta. 243-248 1984).

Results

Sulfhydryl titrations were carried out using the purified rb-bFGF preparation. When a sample, obtained by dialysis of rb-bFGF vs. 0.1 M Tris-HCl, pH 8.0, containing 1.5 mM EDTA, was titrated with DTNB, the mixture showed light scattering rendering the absorbance measurement impossible. Therefore, the dialyzed rb-bFGF sample was diluted with 4 volumes of 6 M guanidinium-HCl (GdnHCl) in 0.1 M Tris-HCl, 1.5 mM EDTA, pH 8.0 immediately followed by DTNB titration. Titration of this sample showed approximately 2.5 moles of free sulfhydryl per molecule.

DTNB titration experiments were also carried out with reduced rb-bFGF. Thus, rb-bFGF in 0.1 M Tris-HCl, 1.5 mM EDTA, pH 8.0 was first reduced with 50 mM DTT. After 2 hours incubation, the reduced rb-bFGF was precipitated with 5% trichloroacetic acid (TCA) and the resultant pellet was washed 3 times with 5% TCA. The final pellet was washed with a small amount of $H_2O$ and the protein solubilized with 6M GdnHCl in 0.1 M Tris-HCl, 1.5 mM EDTA, pH 8.0 immediately followed by DTNB titration. This resulted in detection of approximately 4 moles of free sulfhydryl per mole of reduced rb-bFGF. This result, combined with about 2 detectable free sulfhydryls in non-reduced sample, suggested that the rb-bFGF preparation has two free sulfhydryl groups, and that the other cysteines form a disulfide bond, which can be readily cleaved without denaturants.

Sulfhydryl titrations were also carried out with the S-carboxymethylated (S-CM) rb-bFGF. The S-CM rb-bFGF sample was dialyzed vs. 40 mM Tris-HCl, pH 7.5 and treated with 100 mM DTT overnight. The dialyzed S-CM rb-bFGF was treated with 5% TCA and assayed for free sulfhydryl groups as described above. The results showed 2 moles of free sulfhydryls per mole of S-CM rb-bFGF, indicating that the S-CM rb-bFGF has two free sulfhydryl groups upon reduction. This suggested that rb-bFGF had two free sulfhydryl groups that had been carboxymethylated.

Peptide mapping was employed to investigate whether two cysteines are involved in a disulfide bond and two are in the free reduced state. Digestion with trypsin was used to isolate the cysteines into three peptides: 4-27, containing cys-26; 68-73, containing cys-70; and 88-98, containing cys-88 and cys-93.

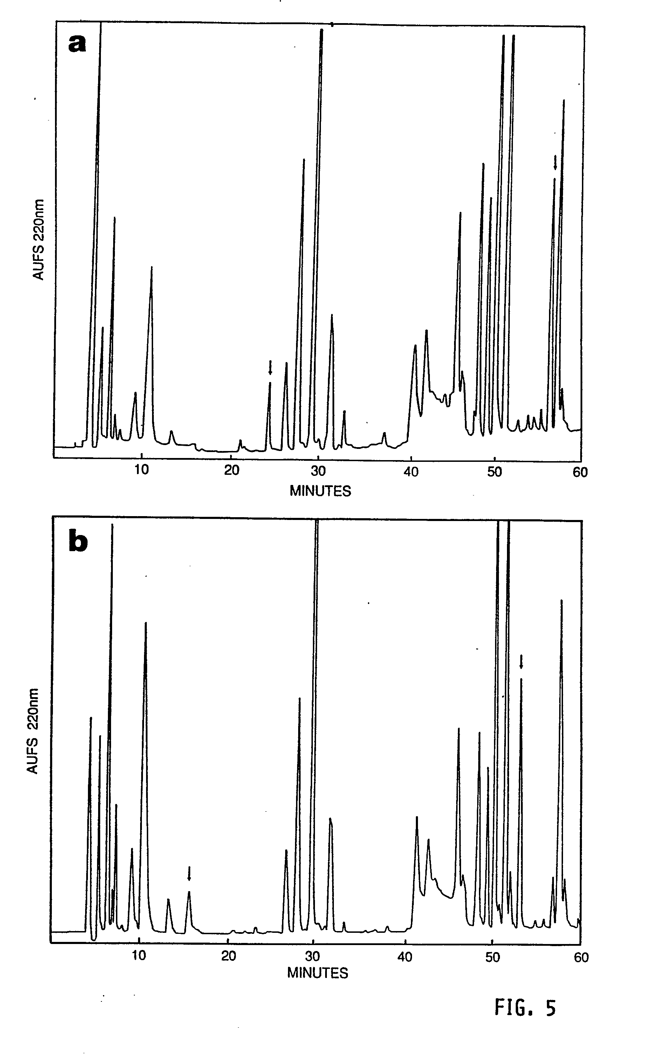

Trypsin digestion was performed on unreduced native rb-bFGF and on unreduced S-CM rb-bFGF. Following digestion, the peptides produced were separated on reverse phase HPLC, and the resulting peptide maps were compared (Figure 5). The introduction of the acetate group into a peptide containing cysteine was predicted to make the peptide more hydrophilic, and hence elute sooner in a reverse phase HPLC gradient.

In Figure 5, it is seen that two peptide peaks are shifted to earlier elution times in the S-CM rb-bFGF relative to the native rb-bFGF. One peak shifts from 25 minutes to 16 minutes and the other from 57 minutes to 53 minutes. These peaks were collected and identified by sequence analysis. The sequence of the peptide eluting at 25 minutes was determined and found to correspond to the tryptic peptide 68-73. This peptide contains cys-70. Since this peptide yielded a single peak, it was presumed to be one of the cysteines not involved in a disulfide bond.

The sequence of the peptide eluting at 53 minutes gave two sequences. Since a single peak in this region shifted on carboxymethylation and the peptide contained within this region gave rise to two sequences, this was assumed to be the peptide containing the disulfide bond. Comparison of the two sequences obtained from the 53-minute peak with the whole FGF sequence indicated that these two constituent sequences correspond to the 24-27 and 88-98 peptides. There are three cysteines in these two peptides. Identification of carboxymethylcysteine phenylthiohydantoin at the first sequence cycle indicated cys-88 as the other free cysteine and suggested that there is a disulfide bond between cysteine 26 and 93.

The S-CM rb-bFGF was assayed in 3T3 cells as described in Example 8. The results showed that the S-CM rb-bFGF has an activity comparable to the native molecule, demonstrating that the free sulfhydryl groups in the molecule are not required for the mitogenic activity on 3T3 cells.

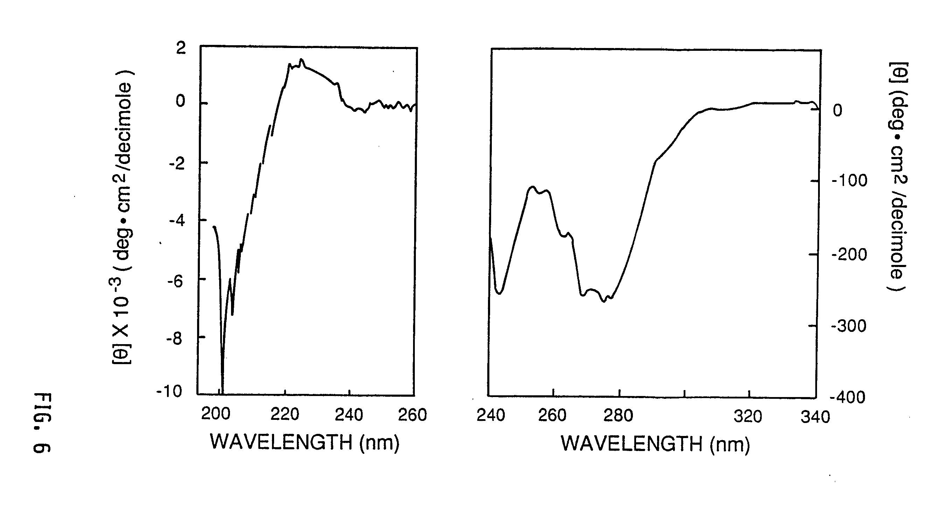

Circular dichroic spectra were obtained with rb-bFGF in 20 mM sodium citrate, 0.1 M NaCl, pH 5.0. The results are shown in Figure 6. The near UV CD shows intense negative ellipticities with a number of extrema. Those extrema at 262 and 269 nm are due to phenylalanine residues, and the extreme at 275 nm probably arises from tyrosine absorbance (Strickland, 2d CRC Crit. Rev. Biochem. 113-175 (1974)). A broad, negative ellipticity can be seen from 306 nm down to 290 nm, which then overlaps with the aromatic CD. This CD band is likely due to a disulfide, which usually has a peak around 240 to 250 nm. Therefore, the observed near UV CD of rb-bFGF may be characterized as a broad negative disulfide CD, and several aromatic CD signals. These results indicate that the aromatic residues are in rigid, asymmetric environments and that the rb-bFGF has a distinct tertiary structure (Timasheff, Vol. II In the Enzymes, Boyer (ed.) 371-443 (1970).

The S-CM rb-bFGF was also examined by CD. The results showed both near and far UV spectra identical to those of the native protein, indicating that the S-carboxymethylation of the two free sulfhydryl groups does not apparently affect the secondary and tertiary structures of the protein. This is consistent with the observation that the S-carboxymethylation does not affect the activity of the protein.

The far UV CD, shown in Figure 6, exhibits a spectrum typical for unordered structure (Greenfield and Fasman, Biochemistry 8, 4108-4116 (1969). The spectrum exhibits a negative peak at 202 nm and a broad positive peak around 225 nm. The 202 nm peak and almost no negative ellipticities between 205 and 220 nm are characteristics typical for unordered secondary structure. The positive peak at 225 nm is likely due to contribution from $\beta$-turns (Chang et al., 91 Anal. Biochem. 13-31 1978). Therefore, the polypeptide of rb-bFGF seems disordered, but folded into a distinct tertiary structure with $\beta$-turns.

Gel filtration of rb-bFGF in 20 mM sodium citrate, 0.1 M NaCl, pH 5.0 showed that the protein elutes at the same elution volume as that for myoglobin (17000). Since the molecular weight of bFGF (16000) is smaller than that of myoglobin, the gel filtration result suggests a slightly asymmetric conformation of the protein.

EXAMPLE 7

HUV-EC Bioassay for rb-bFGF

Human umbilical vein endothelial (HUVE) cells used in this experiment were isolated by Judith A. Berliner by the method of Gimbrone et al, Human Vascular Endothelial Cells in Culture, J. Cell Biol., 60, 673-684 (1974). Cells were maintained by subculturing into culture flasks coated with 0.1% gelatin in phosphate buffered saline, and 10 $\mu$g/ml fibronectin (Boehringer Mannheim, Ingelheim, West Germany) in media minus fetal bovine serum for 30 minutes each, consecutively. Cells were released with 0.0125% trypsin-0.005% EDTA and passed 1:2 or 1:3 once a week. The maintenance media used was MCDB105 (Irvine Scientific, Irvine, California) with penicillin G (10 units/ml) streptomycin (10 $\mu$g/ml), fetal bovine serum (20%, hyclone), L-glutamine (2mM), sodium pyruvate (1mM), heparin (40 $\mu$g/ml, 170 units/mg) and endothelial cell growth supplement (ECGS, 40 $\mu$g/ml Collaborative Research). Cells were grown in a 2% CO2 incubator.

The following experiment was carried out to compare the growth sustaining and mitogenic activities of three different forms of FGF on HUV endothelial cells: 1) E. coli derived recombinant bovine basic FGF (rb-bFGF); 2) bovine acidic FGF (b-aFGF, particularly purified from bovine brain); and, 3) natural bovine basic FGF (bFGF purified from bovine pituitary glands, Sigma Chemical Company, St. Louis, Missouri).

One hundred cells per well were plated into the center 8 wells of four 24-well plates. One of the three fibroblast growth factors identified above was added to each well, except in the case of the control wells, to which no growth factor was added.

The cells were fed three and six days after the initial seeding with their same growth factor. Ten days after the seeding, cultures were analyzed by crystal violet straining.

The results in Table VII illustrate that there were 20% and 32% more colonies in the 8 wells which contained rb-bFGF than colonies in the wells containing either aFGF or natural bFGF, respectively. Also of interest is the fact that 75% of the total colonies arising from culture with rb-bFGF were 0.5 mm and larger, while only 55% and 51% of the colonies grown with aFGF and n-bFGF, respectively were that size.

17

Table VII

| Growth Factor | # of Colonies /8 Wells | # of Colonies ≥ 0.5 mm/8 Wells |
|---|---|---|
| rb-bFGF | 351 | 265 |
| aFGF | 293 | 160 |
| natural bFGF | 265 | 134 |
| Control | 8 | 0 |

EXAMPLE 8

rb-bFGF Bioassay on NIH 3T3 Cells

Cells used for this assay were NIH3T3 cells from ATCC. The cells were grown in DME medium with penicillin G (10 $\mu$g/ml), streptomycin (10 $\mu$g/ml), and calf serum (10%). The cells were passed 1:40, two times a week. On day 1 of the assay, subconfluent cultures were trypsin dispersed and plated into 24-well plates at a concentration of $2 \times 10^4$ cells per ml, 1 ml per well in the above growth media.

On day 5, the media was replaced with DME containing penicillin streptomycin and 5% human platelet poor plasma (cleared heparinized serum), 1 ml per well. On day 6, experimental and control samples of FGF were added to the media in volumes no greater than 100 $\mu$l.

Eighteen hours later, the cells were pulsed for 1 hour with 1 ml of DME containing 5% calf serum and 2 $\mu$Ci of $^3$H-Me thymidine at 37°C. The cells were then washed one time each with 1 ml of phosphate buffered saline (PBS) and 5% trichloroacetic acid, both at 4°C. Plates were allowed to air dry for 30 minutes, after which 1 ml of 0.25 M NaOH was added to each well. After one hour at room temperature, the contents of each well were transferred to a separate counting vial containing 10 ml of Aquasol® II (New England Nuclear, Boston, Massachusetts). Samples were counted for 1 minute through the 0-397 window of a Beckman LS 7,500 scintillation counter (Beckman Instruments, Inc., Fullerton, California).

Recombinant b-bFGF standards were made from a stock with a concentration of 600 $\mu$g/ml in a sodium citrate buffer of pH 5. The range of standards used was 5 pg to 1,000 pg per ml. The standard of lowest concentration which gave maximal $^3$H-thymidine uptake was 500 pg. An average of 140-210 pg gave the half maximal incorporation of labelled thymidine.

Example 9

Construction of the rh-bFGF gene

Conversion of the bovine bFGF gene prepared in Example 1 to a gene encoding for h-bFGF was accomplished by oligo site directed mutagenesis.

The segment to be modified was first cloned into the phage vector M13mp18 and transformed into E. coli JM101 for growth and preparation of single stranded phage DNA (Messing, J. Vol. 9, Nucl. Acid Res., 309-321 (1981)). Approximately 0.5 $\mu$g of template DNA was mixed with 5 pmol of universal M13 sequencing primer and 5 pmol of each mutagenic primer, heated to 65°C for 3 minutes and allowed to slow cool. The annealed template-primer was then mixed with ATP, a deoxynucleotide triphospate (dNTP) mix, DNA polymerase I (DNA PolI) large fragment (Klenow fragment), and ligase followed by incubation at 15°C for 4 hours. Aliquots of this reaction mix were transformed into competent E. coli JM101 cells and plated in 0.7% Luria agar.

Plaques containing mutant phage were selected by hybridization of replica nitrocellulose filters with $^{32}$P-labeled mutagenic primer. Single strand DNA was prepared from positive-screening plaques and its sequence verified using the dideoxy chain-termination method. The amino acid changes made and the corresponding mutagenic primers used were:

pro-129 to ser-129) 5′ GACCAGTCTTCGAACCCAGTTTGTA 3′
ser-113 to thr-113) 5′ CATACCAGGAAGTGTATTTACGAGA 3′
    The primers correspond to the antisense strand of the bFGF gene.


Example 10


Purification of rh-bFGF

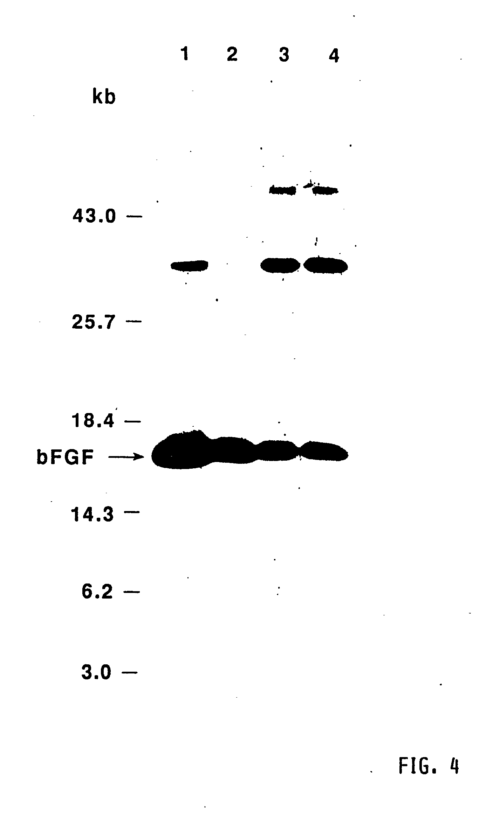

    E. coli cells containing the synthetic h-bFGF genes in pCFM1156 were grown as described above in Example 4. After disruption of the cells and low speed centrifugation, the resultant rh-bFGF (Table VI) was found both in the supernatant and pellet fractions. Purification from the pellet requires solubilization by denaturants followed by refolding to obtain active material. These steps can be avoided by purification from the supernatant fraction as described below. This fraction was applied to a CM-sepharose column in 40 mM Tris-HCl, pH 7.4 and eluted with a linear NaCl gradient. The fractions containing rh-bFGF were then bound to the same resin, but in 40 mM Tris-HCl, pH 8.2 and again eluted with a linear NaCl gradient. In these two chromatographies, 1 mM DTT was included to prevent oxidation, which otherwise resulted in the formation of intermolecular disulfide bonds. The protein was further purified by gel filtration using a Sephadex® G-75 column (Pharmacia, Upppsala, Sweden) in 20 mM sodium citrate, 0.1 M NaCl, pH 5.0. Initial attempts to purify rb-bFGF from E. coli cells showed that dimer was readily formed when 1 mM DTT was not included throughout the purification process. Purification of the human bFGF was carried out in essentially the same manner as for the bovine material as set forth in Example 4. No difference between rh-bFGF and rb-bFGF was noted in any of the purification steps. When examined on SDS-PAGE under reducing conditions, the r-bFGFs gave a major band at 16,500 daltons corresponding to the monomeric molecular weight and minor bands at higher molecular weights which probably represent dimer and tetramer forms. (See Fig. 4.) When run under non-reducing conditions, more contamination by the higher molecular weight bands was apparent. Amino acid sequence analysis of the purified rh-bFGF revealed that methionine had been cleaved from most of the material, yielding 70% proline, 13% alanine, and only 17% methionine on the N-terminus. As does the natural bFGF, rh-bFGF exhibits a strong affinity for heparin, eluting from heparin sepharose columns at approximately 1.5-2.0 M NaCl (data not shown).


Example 11


Characterizations of rh-bFGF

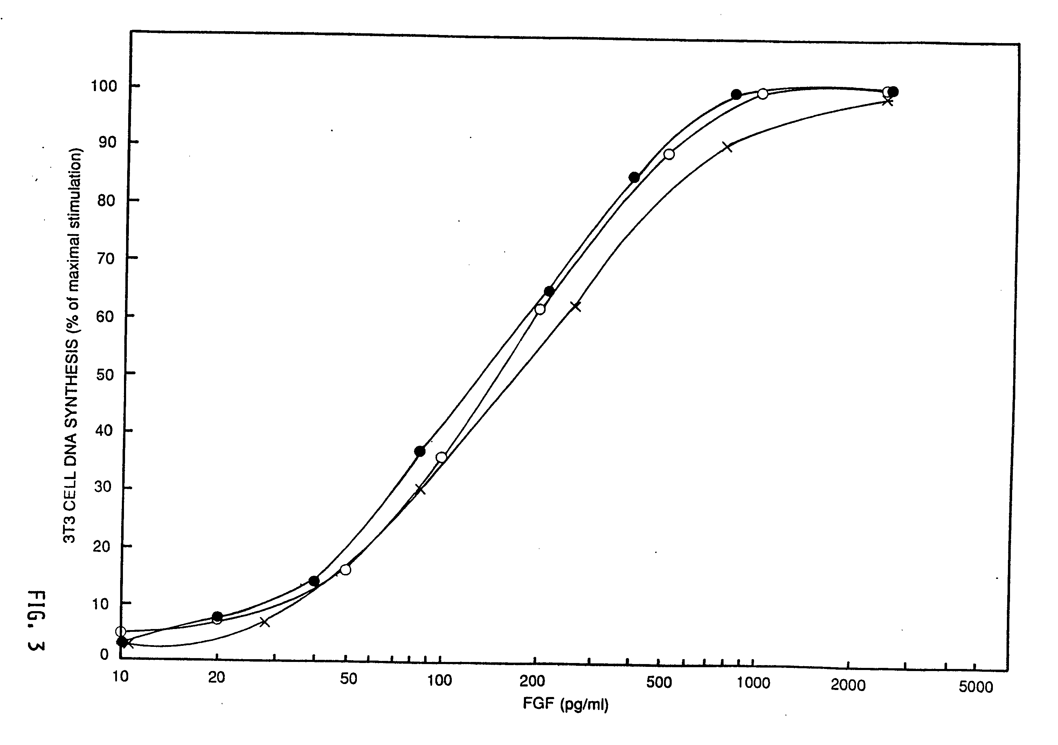

    The activity of rh-bFGF was examined in $^3$H-thymidine incorporation on 3T3 cells as described in Example 8. As shown in Fig. 3, rh-bFGF shows essentially identical potency, in this assay, as rb-bFGF, producing half-maximal stimulation of DNA synthesis at a dose of about 150-200 pg/ml.


Example 12


HUV-EC BioAssay for rh-bFGF

    The HUVE cell assay is described in Example 7, with minor variations in the plating and time of assay. Addition of rh-bFGF resulted in extensive cell proliferation in comparison with controls containing no growth factor. No significant difference between recombinant bovine and human bFGF was observed.

Table VIII

| Growth Factor | Total Colonies | Large Colonies |
|---|---|---|
| rh-bFGF | 76 | 22 |
| rb-bFGF | 68 | 19 |
| Control | 23 | 0 |

All fibroblast growth factors were added at a concentration of 10 ng/ml at the time of plating and on days 3 and 6 after plating. Cells were stained and counted on day 9. Colonies greater than 0.5 mm in diameter were scored as large colonies.

Example 13

Preparation of rh-bFGF Analogs

In order to improve stability and facilitate purification of rh-bFGF, oligonucleotide site-directed mutagenesis was used to modify the human bFGF gene so that the sequences which coded for some or all of the cysteine residues would code for serine instead. It is recognized that the bovine bFGF gene can be modified in the same manner and that the sequences which coded for one or all of the cysteine residues could code for other amino acids, such as, alanine, aspartic acid and asparagine. Four oligonucletoides were synthesized, each designed as shown in the table below:

Table IX

| Oligonucleotide | Sequence | coding change |
|---|---|---|
| 102-21 | 5′ ACCGTTTTTGGAGTACAGACG 3′ | CYS TO SER AT POSITION 26 |
| 102-22 | 5′ CCGGTTAGCAGATACACCTTT 3′ | CYS TO SER AT POSITION 70 |
| 102-23 | 5′ GTCAGTAACAGACTTAGAAGC 3′ | CYS TO SER AT POSITION 88 |
| 102-24 | 5′ GAAAAAGAAAGATTCGTCAGT 3′ | CYS TO SER AT POSITION 93 |

The first mutagenesis used oligonucleotides 102-22 and 102-23 to convert the cysteines at positions 70 and 88 to serine. Subsequently, genes were constructed in which all 6 possible pairs of cysteines are replaced by serines and one in which all 4 cysteines are replaced by serines. Oligonucleotides used in the construction of these mutant genes are listed below:

Table X

| Analog | oligonucleotides used for mutagenesis |
|---|---|
| serine 70,88 | 102-22, 102-23 |
| serine 26,93 | 102-21, 102-24 |
| serine 26,70, | 102-21, 102-22 |
| serine 26,88 | 102-21, 102-23 |
| serine 70,93 | 102-22, 102-24 |
| serine 88,93 | 102-23, 102-24 |
| serine 26,70,88,93 | 102-21, 102-22, 102-23, 102-24 |

All mutagenesis reactions were carried out in essentially the same manner, differing only in the oligonucleotides used for the reaction and for screening of the resultant plaques by hybridization. Ten pmole each of the M13 universal primer and the above primers were phosphorylated by incubation with 1 mM ATP and 10 units of polynucleotide kinase in 10 $\mu$l of 70 mM tris, 10 mM MgCl2' 5 mM DTT for 30 minutes at 37°C. Five pmole of each kinased oligonucleotide was mixed with about 0.5 $\mu$g of single stranded M13mp18/rb-bFGF, heated to 60°C, and allowed to renature by cooling to room temperature. To this template/primer mix was then added 1 $\mu$l of a solution 25 mM each in dATP, dCTP, dGTP, and TTP, 1 $\mu$l of 100 mM ATP, 2 units T4 ligase, and 8 units DNA PolI large fragment. This mixture was incubated at 14°C for 4 hours. Aliquots of the ligation mix were transformed into E. coli JM101 made competent by treatment with 50 mM CaCl2 and plated in 0.7% Luria agar on previously poured 1.5% Luria agar plates. Lifts onto nitrocellulose filters were performed on the resulting clear plaques and filters were screened by hybridization to the appropriate radiolabeled oligonucleotide. Several positives were obtained using each probe. Positive plaques were picked, and single strand DNA was prepared for use as a template in the second round of mutagenesis. When the desired construction was obtained, replicative form DNA was purified by cell lysis and banding in a CsCl density gradient. The modified genes were then transferred to the plasmid vector pCFM1156 for expression in E. coli. The modified gene was excised from its M13 vector by cleavage with XbaI and HindIII and purified by agarose gel electrophoresis. This purified fragment was then ligated into XbaI/HindIII cut pCFM1156. The new constructions were transformed into E. coli strain FM5 for expression. The serine-70,88 and serine-26,93 analogs were transferred to the expression vector.

Growth of the production strain and subsequent purification of the serine-70,88 analog was carried out exactly as for the rb-bFGF and rh-bFGF as described in Example 4, except that DTT was omitted from all purification steps. The omission of DTT was possible since the cysteine residues responsible for dimerization of the bFGF during purification had been removed. Thus, the serine-70,88 analog represents a significant improvement over the r-bFGF with the natural sequence since it contains no detectable dimer impurity and will not tend to form dimer over time because it lacks free sulfhydryl groups. Further, since the need for added reducing agent is eliminated, possible formulation problems may be avoided.

The serine-26,93 analog did not behave in the same manner as the recombinant bovine and human bFGF and serine-70,88 molecules, probably because it does not have the disulfide structure of natural bFGF. During purification a significant portion of the serine-26,93 analog was degraded. Such degradation also occured when attempts were made to purify the rb-bFGF in the presence of 7M urea, which denatures the molecule's tertiary structure. Although the serine-26,93 analog does not appear to have the same biological activity as the serine-70,88 analogs when purified from the supernatant as specified in Examples 4 and 11, the inactive analogs may have application as antagonists or blocking molecules.

Example 14

Purification of a serine-26,70,88,93 analog from inclusion bodies

It was surprisingly found that when a serine-26,70,88,93 analog was purified from inclusion bodies, rather than from the supernatant, an active analog was obtained, despite alteration of apparent non-free sulfhydryls.

Using oligo site-directed mutagenesis, the synthetic gene for h-bFGF was modified generally as described in Example 9 to replace all four cysteine codons with nucleotides coding for serine residues. The corresponding protein was expressed in E. coli and purifed from inclusion bodies by solubilization in urea followed by a series of column chromatographies and a folding step. The resulting protein, having no cysteine residues, is unable to form either intramolecular or intermolecular disulfide bonds. Nevertheless, this analog protein was found to exhibit mitogenic activity on NIH 3T3 cells indistinguishable from that exhibited by the natural sequence.

Oligo site-directed mutagenesis

A previously constructed bFGF analog gene in which codons for cysteines 70 and 88 had been converted to serine codons was used as the starting template for the mutagenesis. Approximately 0.5 $\mu$g of template DNA was mixed with 5 pmol universal M13 sequencing primer and 5 pmol of each mutagenic primer, heated to 65°C for 3 minutes and allowed to slow cool. The annealed template-primer was then mixed with ATP, a dNTP mix, DNA PolI large fragment, and ligase, followed by incubation at 15°C overnight. Aliquots of this reaction mixture were transformed into competent JM101 cells and plated in 0.7% Luria agar. Plaques containing mutant phage were selected by hybridization of replica nitrocellulose filters with each $^{32}$P-labeled mutagenic primer. Single strand DNA was prepared from plaques which scored positive in both hybridization screens. Selection of the desired sequence was verified using the dideoxy chain-termination DNA sequencing method. The amino acid changes made and the corresponding mutagenic primers used were:

cys-26 to ser-26 5′ ACCGTTTTTGGAGTACAGACG 3′

cys-93 to ser-93 5′ GAAAAAGAAAGATTCGTCAGT 3′

Both primers correspond to the antisense strand of the bFGF gene.

## Expression and Purification.

E. coli cells harboring the plasmid containing the mutant bFGF gene were grown in 2x Luria broth at 30°C to approximately 0.5 A600, than shifted to 42°C to induce expression of bFGF. After growth overnight, the cells were harvested by centrifugation and lysed by three passages through a French Press at 10,000 psi. The insoluble material containing the bFGF trapped in inclusion bodies was collected by low speed centrifugation. The inclusion bodies were solubilized in urea and subjected to cation exchange and silica column chromatographies. The partially purified analog was folded by dilution and then purified to near homogeneity by cation-exchange column chromatography. Recombinant natural sequence human bFGF was purified from the soluble fraction of lysed E. coli cells by a series of chromatographic steps.

## Mitogenesis Assay on NIH 3T3 Cells

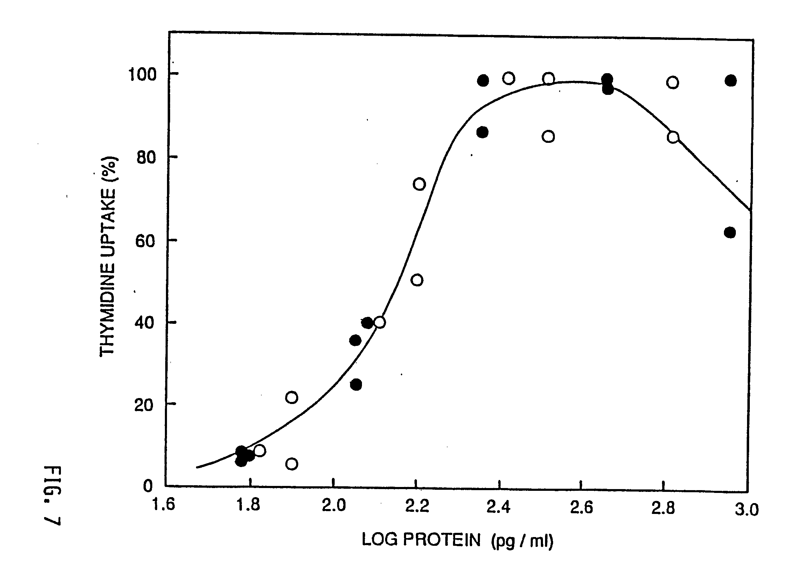

The biological activity of the analog bFGF was tested by its ability to stimulate $^3$H-thymidine uptake in confluent cultures of NIH 3T3 cells, as described in Example 7. Figure 7 shows the concentration dependence of mitogenic acitivity for the serine-26,70,88,93 analog and for natural sequence h-bFGF. Within experimental error, the two profiles are indistinguishable, with the half-maximal mitogenic effect observed at a dose of about 150 pg/ml. These results indicate that the conformation required for receptor-mediated mitogenic acitivity is not altered by the substitution of the four cysteine resides, at least where the analog is purified from inclusion bodies, rather than from the supernatant.

## Example 15

## Bioactivity of rh-bFGF analogs

The biological activity of the rh-bFGF analogs was characterized as described for the bovine form in Examples 7 and 8. The half-maximal activity of the rh-bFGF serine-70,88 analog in the mitogenic assay on NIH3T3 cells (example 8) is identical to that of the rb-bFGF and rh-bFGF as shown in Figure 3. The serine-26,93 analog showed no activity in this assay, as expected.

The serine-70,88 analog was also tested for its ability to support the growth of HUVE cells as described in Example 7. The rh-bFGF serine-70,88 analog was equally as effective as the rb-bFGF and rh-bFGF in promoting the growth of HUVE cells in culture.

### Table XI

| Growth Factor | Total Colonies | Large Colonies |
|---|---|---|
| Ser-70,88 rh-bFGF | 84 | 24 |
| rh-bFGF | 76 | 22 |
| rb-bFGF | 68 | 19 |
| Control | 23 | 0 |

## Example 16

In vivo study of efficacy of serine-70,88 analog

### Pre-operative Preparation

New Zealand White rabbits, weighing approximately 3 kg each were anesthetized using 5 mg/kg Rompun® (Farbenfabriken, Bayer, West Germany) as a sedative, followed (10 minutes later) by approximately 50-60 mg/kg ketamine, both administered intramuscularly. Each rabbit's weight was measured and recorded. A small cotton or gauze plug was inserted into both ears of each rabbit, after which the inner surface and outer edges of both ears were shaved using an animal clipper (#40 blade). Commercially available Neet® depilatory cream was then applied to the inner surface of each ear for 10 minutes, after which time it was removed with dry gauze. The inner surface of the ears was wiped with saline-soaked gauze followed by application of a 70% alcohol solution. The dermis of the inner surface of one ear of each rabbit was blanched by infiltration of the ear with a 2% xylocaine solution containing 1:1000 epinephrine (this requires 1.5-3 cc total volume) using a 30 gauge needle. The infiltrated area was then scrubbed with 3 cycles of betadine followed by the 70% alcohol solution. Where necessary, the ear plugs were replaced with dry plugs at this point.

The rabbits were then transferred to a sterile surgical room. The blanched ear was immobilized on a plexiglass "ear board" (Washington University Medical Center, Division of Technical Services) which utilizes two bar clamps, one at the tip and one at the base of the animal's ear, to stabilize the rabbit ear without compromising its blood supply. The animal was draped, and the surgical field (i.e., the inner surface of the blanched ear) sprayed with Betadine and allowed to dry for 3-5 minutes.

### Wounding

Sterile technique was employed throughout the wounding procedure. The surface of the inner ear was scored gently with a 6 mm biopsy punch, and the biopsy site cleared of all tissue and fibers (including the periosteal membrane) down to the level of bare cartilage, using micro-surgical forceps, tenotomy scissors, a blunt edged 2 mm Lempert periosteal elevator, and sterile cotton-tipped applicators. Biopsies in which the cartilage was completely cut through by the punch were not used for experimental purposes. However, partial thickness scores of the cartilage were considered acceptable. The location of any nicks or natural holes in the cartilage was carefully noted and recorded (for reference on the harvest day). Blood was removed from the biopsy site with sterile, cotton-tipped applicators, with care taken to avoid excess blood in the wound. Each completed biopsy was covered with a small piece of saline-soaked gauze. Four viable biopsies were placed on each wounded ear, two on each side of the midline (as defined by the fold in the ear when it was stabilized upon the board. In any event, no more than 5 total biopsies were placed on each ear. The biopsies were positioned a minimum of 1 cm apart.

Upon completion of one ear, the ear was covered with saline-moistened gauze and then taped shut around the gauze to retain moisture until application of FGF. The second ear was then blanched, scrubbed, immobilized, and wounded in the same manner as the first ear. Blood was removed from the biopsy site of each second ear and each completed biopsy covered with a small piece of saline-soaked gauze. Upon completion of the second ear, it also was covered with saline-moistened gauze until application of FGF. Any rabbit that showed evidence of recovery from anesthesia at any time prior to this point in the procedure was reanesthetized with 25 mg/kg ketamine, administered intramuscularly.

### Application of FGF Preparations

FGF was applied first to the first wounded ear, with the moist gauze being removed from the first ear only and the unwounded surfaces of the ear gently wiped dry with gauze. All biopsies were gently cleared of any blood or excess fluid with sterile, cotton-tipped applicators. The unwounded surfaces of the ear were painted with tincture benzoin compound, carefully avoiding the biopsies, and allowed to air dry for 3-5 minutes.

5 µg of either rb-bFGF or ser-70,88 rh-bFGF in Zyderm® (Collagen Corporation) was applied to each biopsy using a 26 gauge needle permanently mounted on a low dead space 0.5 cc or 1 cc syringe (Becton-Dickinson), or a micropipetter. The biopsy was able to accomodate a maximum of 0.025 cc of the viscous bFGF-Zyderm® preparation. After applying FGF to one biopsy, it was immediately covered with the occlusive dressing Tegaderm® (3M Corporation, Minneapolis, Minnesota), being careful not to form any air bubbles or wrinkles over the biopsy site. Tegaderm® was precut to an approximate size of 2 cm². This process was repeated for each biopsy on the ear. Any failed biopsies were also covered with Tegaderm® to minimize risk of infection. Following completion of the first ear, the moist gauze was removed from the remaining ear, and the procedure repeated, making sure to gently clear all biopsies of any blood or excess fluid with sterile, cotton-tipped applicators.

The rabbits were allowed to recover from anesthesia under the observation of the investigator performing the surgery. Upon recovery, a plastic collar extending approximately 15-25 cm outward was placed around each rabbit's neck, to prevent the rabbit from disrupting the wounds or dressings. The rabbits were returned to an isolation cage where they were maintained until harvest. The wounds of any rabbits which had removed their collars, and any wound on which the Tegaderm® had been disrupted in some way prior to the harvest date, were re-evaluated as soon as the problem was noted, and discarded from analysis if the wounds appeared to be damaged.

Harvest

On the seventh day post-wounding, the rabbits were anesthetized in the same manner as described for pre-operative preparation. Each rabbit's weight was measured and recorded, and a qualitative description of the condition of the wounds was recorded, noting in particular the presence or absence of the Tegaderm® and of any excess fluid under the dressing. The rabbits were sacrificed with 50 cc/kg air embolism administered by intracardiac injection; both ears were then amputated from the body using a #15 surgical blade mounted on a knife handle.

Each biopsy, with approximately 5 mm of surrounding tissue on any side and the Tegaderm® still intact, was excised from the ear, and the biopsy site measured in order to bisect it accurately at the midline, making reference to notes taken on the day of wounding to avoid bisecting through natural holes or nicks in the cartilage. The biopsy was carefully bisected with a single edge razor blade, using a single downward motion to avoid disrupting the wound orientation. The bisected biopsies were immediately placed in cassettes labeled with the rabbit identification number, and placed in the appropriate fixative for subsequent histologic processing and quantitative analysis.

Quantitative Histological Analysis

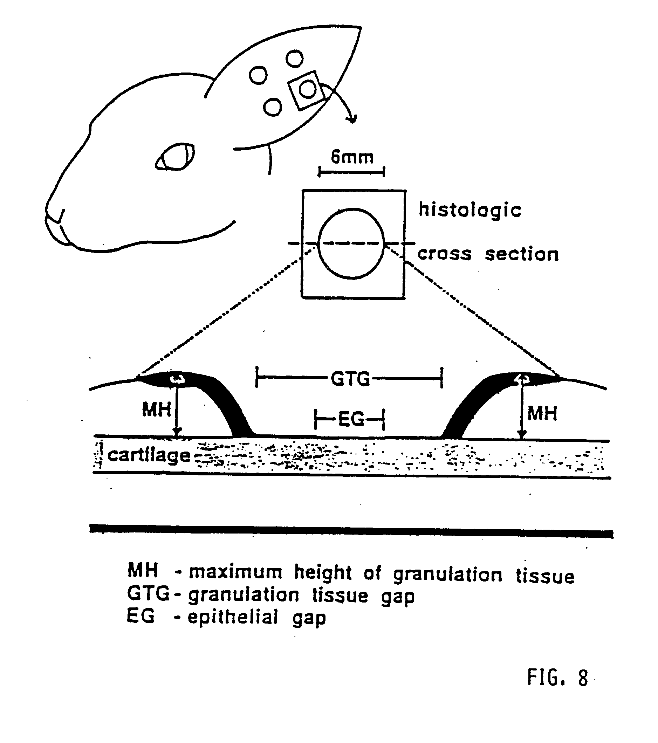

Carefully oriented cross sections through bisected wounds were embedded, sectioned, and stained, using either a mixture of Hematoxylin and Eosin or Trichrome. Rough cutting of the section was minimized in order to obtain a cross section thrugh the true wound center. Measurements made include the reepithelialization gap (EG) across the wound, the maximum height (MH) of granulation tissue at the wound at the wound edges (the average of both sides), and the granulation tissue gap (GTG) across the wound, as shown in Fig. 8. Measurements were made blindly on precoded slides by two independent observers using a calibrated lens micrometer and converting to millimeters (mm).

The average of both observers' measurements were calculated, after which the code was broken and the data statistically analyzed. If there was greater than 10% difference between observers, the slide was reanalyzed. The resulting data is shown in Table XII.

Table XII

| rb-bFGF | | |
|---|---|---|
| | Control | rb-bFGF |
| Number of wounds | 41 | 22 |
| Frequency of total reepithelialization | 24% | 50% |
| GTG | 4.68±0.12 | 4.66±0.13 |
| MH of new granulation tissue (mm) | 0.76±0.03 | 0.79±0.02 |
| ser-70, 88 rh-bFGF analog | | |
| | Control | ser(70,88) rh-bFGF |
| Number of wounds | 126 | 13 |
| Frequency of total reepithelialization | 22% | 69% |
| GTG | 4.41±0.09 | 3.85±0.14 |
| MH of new granulation tissue (mm) | 0.60±0.01 | 0.74±0.04 |

The data is presented as the mean ± standard error.

Both the rb-bFGF and ser-70,88 rh-bFGF in Zyderm® preparations had a significant positive effect on reepithelialization, although the effect with ser-70,88 rh-bFGF was somewhat greater. However, the formulation of new granulation tissue was significantly affected by ser-70,88 rh-bFGF, while rb-bFGF appeared to have no effect. A possible explanation for this difference is the enhanced stability of the ser-70,88 rh-bFGF analogue compared to the natural sequence material. Since the FGF is applied only once in this study, i.e., at the time of wounding, stability in Zyderm® collagen could be critical to the production of the positive effect on granulation tissue formation. Other wound healing and surgical applications using the bFGF analogs of the present invention will be apparent to those skilled in the art.

Numerous modifications and variations in the practice of the invention will also be apparent to those skilled in the art upon consideration of the foregoing illustrative .examples. Consequently, the invention should be considered as limited only to the extent reflected by the appended claims.

The features disclosed in the foregoing description, in the following claims and/or in the accompanying drawings may, both separately and in any combination thereof, be material for realising the invention in diverse forms thereof.

## Claims

1. An analog of basic fibroblast growth factor which differs from naturally occurring basic fibroblast growth factor in terms of the identity and/or location of one of more amino acid residues, wherein at least one of the cysteine residues of said naturally occurring basic fibroblast growth factor is is replaced by a residue of a different amino acid.

2. The analog of Claim 1 wherein said different amino acid is selected from the group consisting of serine, alanine, aspartic acid and asparagine.

3. The analog of Claim 2 wherein, said different amino acid is serine.

4. The analog of Claim 1 wherein at least one of said replaced cysteine residues is a cysteine residue existing as a free sulfhydryl.

5. The analog of Claim 1 wherein two of said cysteine residues are replaced by a residue of a different amino acid.

6. The analog of Claim 5 wherein two of said replaced cysteine residues are cysteine residues existing as free sulfhydryls.

7. An analog of basic fibroblast growth factor having the amino acid sequence set forth in Table VI and allelic variants thereof, wherein at least one of the cysteine residues at positions 26, 70, 88, and 93, is replaced by a residue of a different amino acid.

8. The analog of Claim 7 wherein at least one terminal amino acid residue is deleted while said analog substantially retains the biological activity of naturally occurring basic fibroblast growth factor.

9. The analog of Claim 7 wherein said different amino acid is selected from the groups consisting of serine, alanine, aspartic acid, and asparagine.

10. The analog of Claim 8 wherein said different amino acid is serine.

11. The analog of Claim 7 wherein at least one of said replaced cysteine residues is selected from the group consisting of cysteines at amino acid positions 70 and 88.

12. The analog of Claim 7 wherein two of said cysteine residues is replaced by a residue of a different amino acid.

13. The analog of Claim 11 wherein said replaced amino acids comprise cysteines at amino acid positions 70 and 88.

14. The analog of Claim 11 wherein said replaced cysteine residues comprise cysteines at amino acid positions 26 and 93.

15. The analog of Claim 12 wherein said replaced cysteine residues comprise cysteines at amino acid positions 26, 70, 88, and 93.

16. A DNA sequence encoding for procaryotic or eucaryotic expression of an analog of a basic fibroblast growth factor of Claim 7.

17. The DNA sequence of Claim 16 wherein said DNA sequence is modified from human fibroblast growth factor gene.

18. The DNA sequence of Claim 17 wherein said human basic fibroblast growth factor gene is modified by oligonucleotide site-directed mutagenesis.

19. The DNA sequence of Claim 18 wherein said human basic fibroblast growth factor is modified from a bovine basic fibroblast growth factor gene.

20. The DNA sequence of Claim 19 wherein said bovine fibroblast growth factor gene is modified by oligonucleotide site-directed mutagenesis.

21. A pharmaceutical composition comprising a therapeutically effective amount of a basic fibroblast growth factor analog according to Claim 1 and pharmaceutically acceptable adjuvants.

22. A method for treating a wound comprising administering to said wound a therapeutically effective amount of a basic fibroblast growth factor analog according to Claim 1.

23. The method of Claim 22 wherein said wound is a surface wound.

24. The method of Claim 22 wherein said wound is a surgical wound.

25. The method of Claim 22 wherein said wound includes a bone fracture or defect.

26. The method of Claim 22 wherein said wound includes a damaged nerve.

27. A method for generating tissue and/or organs comprising administering a therapeutically effective amount of a basic fibroblast growth factor analog according to Claim 1.

28. A procaryotic or eucaryotic host cell transformed or transfected with DNA according to Claim 16 in a manner allowing the host cell to express a basic fibroblast growth factor analog of Claim 7.

29. A method of producing a purified and isolated basic fibroblast growth factor analog comprising the steps of:
transfecting or transforming host cells with DNA according to Claim 16;
culturing the transfected or transformed host cells to allow the host cells to express a basic fibroblast growth factor analog; and,
isolating said basic fibroblast growth factor analog.

30. A method for the purification of a recombinant basic fibroblast growth factor analog according to Claim 7 comprising subjecting a supernatant containing basic fibroblast growth factor analog to non heparin chromatography.

31. A method for the purification of a recombinant basic fibroblast growth factor analog according to Claim 7 comprising the steps of:
solubilizing the inclusion bodies from host cell cultures containing basic fibroblast growth factor; and,
subjecting the solubilized inclusion bodies to non heparin chromatography.

FIG. 1

```
                                      1
                              Met Pro Ala Leu Pro Glu Asp Gly Gly
        TCTAGAAGGAGGAATAACAT ATG CCA GCT CTG CCA GAA GAT GGT GGA
          Xba I

        10                                              20
        Ser Gly Ala Phe Pro Pro Gly His Phe Lys Asp Pro Lys Arg Leu
        TCC GGT GCT TTC CCG CCA GGT CAT TTC AAA GAT CCG AAA CGT CTG

                                    30
        Tyr :Cys: Lys Asn Gly Gly Phe Phe Leu Arg Ile His Pro Asp Gly
        TAC :TGC: AAA AAC GGT GGT TTT TTC CTG CGT ATC CAT CCG GAT GGT
            : C :
        40 :Ser:                                        50
        Arg Val Asp Gly Val Arg Glu Lys Ser Asp Pro His Ile Lys Leu
        CGT GTT GAT GGT GTA CGT GAG AAA TCT GAT CCG CAT ATC AAA CTG

                                    60
        Gln Leu Gln ala Glu Glu Arg Gly Val Val Ser Ile Lys Gly Val
        CAG CTG CAA GCT GAA GAG CGT GGT GTA GTT TCT ATT AAA GGT GTA

        :70                                             80
        :Cys: Ala Asn Arg Tyr Leu Ala Met Lys Glu Asp Gly Arg Leu Leu
        :TGT: GCT AAC CGG TAC CTG GCT ATG AAA GAA GAC GGT CGT CTG CTG
        : C :          Kpn  I
        :Ser:

                        .......  90
        Ala Ser Lys :Cys: Val Thr Asp Glu :Cys: Phe Phe Phe Glu Arg Leu
        GCT TCT AAG :TGT: GTT ACT GAC GAA :TGT: TTC TTT TTC GAA CGT CTG
                    : C :                 : C :
                    :Ser:                 :Ser:

        100                                             110
        Glu Ser Asn Asn Tyr Asn Thr Tyr Arg Ser Arg Lys Tyr |Ser| Ser
        GAA TCT AAC AAC TAC AAC ACT TAC AGA TCT CGT AAA TAC |TCT| TCC
                                                            |A  |
                                                            |Thr|

                                    120
        Trp Tyr Val Ala Leu Lys Arg Thr Gly Gln Tyr Lys Leu Gly |Pro|
        TGG TAT GTA GCT CTG AAA CGT ACT GGT CAG TAC AAA CTG GGT |CCG|
                                                                |T  |
                                                                |Ser|

        130                                             140
        Lys Thr Gly Pro Gly Gln Lys Ala Ile Leu Phe Leu Pro Met Ser
        AAG ACT GGT CCG GGT CAG AAA GCT ATC CTG TTT CTG CCG ATG TCT

                141
        Ala Lys Ser End End
        GCT AAA TCT TAA TAG CTCGAGAAGCTT
                              Hind III
```

FIG. 2

FIG. 3

FIG. 4

EP 0 320 148 A1

FIG. 5

FIG. 6

FIG. 7

MH  - maximum height of granulation tissue
GTG- granulation tissue gap
EG  - epithelial gap

FIG. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | PROCEEDINGS OF THE NATIONAL ACADEMY OF SCIENCES USA, vol. 82, October 1985, pages 6507-6511; F. ESCH et al.: "Primary structure of bovine pituitary basic fibroblast growth factor (FGF) and comparison with the amino-terminal sequence of bovine brain acidic FGF" <br> --- | | C 07 K 13/00 <br> C 12 N 15/00 <br> C 12 P 21/02 // <br> C 12 N 1/20 <br> C 12 N 5/00 |
| A | EMBO JOURNAL, vol. 5, no. 10, October 1986, pages 2523-2528, IRL Press Ltd; J.A. ABRAHAM et al.: "Human basic fibroblast growth factor: nucleotide sequence and genomic organization" <br> --- | | |
| P,A | PROCEEDINGS OF THE NATIONAL ACADEMY OF SCIENCE USA, vol. 85, April 1988, pages 2324-2328; A. BAIRD et al.: "Receptor- and heparin-binding domains of basic fibroblast growth factor" <br> ----- | | |

**TECHNICAL FIELDS SEARCHED (Int. Cl.4)**

C 07 K 13/00
C 12 P 21/00
C 12 N 15/00
A 61 K 37/00

The present search report has been drawn up for all claims

| Place of search | Date of completion of the search | Examiner |
|---|---|---|
| THE HAGUE | 22-03-1989 | NOVOA Y SANJURJO M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document